(19) Europäisches Patentamt
European Patent Office
Office européen des brevets

(11) EP 2 264 174 A2

(12) EUROPEAN PATENT APPLICATION

(43) Date of publication:
**22.12.2010 Bulletin 2010/51**

(21) Application number: **10156270.0**

(22) Date of filing: **21.07.1999**

(51) Int Cl.:
***C12N 15/12*** *(2006.01)* ***C07K 14/47*** *(2006.01)*
***C07K 16/18*** *(2006.01)* ***G01N 33/68*** *(2006.01)*
***C12Q 1/68*** *(2006.01)*

(84) Designated Contracting States:
**AT BE CH CY DE DK ES FI FR GB GR IE IT LI LU MC NL PT SE**

(30) Priority: **22.07.1998 GB 9816024**
**19.07.1999 GB 9916898**

(62) Document number(s) of the earlier application(s) in accordance with Art. 76 EPC:
**99934913.7 / 1 098 972**

(27) Previously filed application:
**21.07.1999 PCT/GB99/02360**

(71) Applicant: **SmithKline Beecham Limited**
**Middlesex TW8 9GS (GB)**

(72) Inventors:
- **Michalovich, David**
**Harlow**
**Essex CM17 5AW (GB)**
- **Prinjha, Rabinder Kumar**
**Harlow**
**Essex CM19 5AW (GB)**

(74) Representative: **Sayce, Alastair George et al**
**GlaxoSmithKline**
**Corporate Intellectual Property (CN925.1)**
**980 Great West Road**
**Brentford, Middlesex TW8 9GS (GB)**

Remarks:
This application was filed on 11-03-2010 as a divisional application to the application mentioned under INID code 62.

(54) **Protein similar to neuroendocrine-specific protein, and encoding CDNA**

(57) MAGI polypeptides and polynucleotides and methods for producing such polypeptides by recombinant techniques are disclosed. Also disclosed are methods for utilizing MAGI polypeptides and polynucleotides in diagnostic assays.



Printed by Jouve, 75001 PARIS (FR)

## Description

### Field of the Invention

This invention relates to newly identified polypeptides and polynucleotides encoding such polypeptides, to their use in diagnosis and in identifying compounds that may be agonists, antagonists that are potentially useful in therapy, and to production of such polypeptides and polynucleotides.

### Background of the Invention

The drug discovery process is currently undergoing a fundamental revolution as it embraces "functional genomics", that is, high throughput genome- or gene-based biology. This approach as a means to identify genes and gene products as therapeutic targets is rapidly superceding earlier approaches based on "positional cloning". A phenotype, that is a biological function or genetic disease, would be identified and this would then be tracked back to the responsible gene, based on its genetic map position.

Functional genomics relies heavily on high-throughput DNA sequencing technologies and the various tools of bioinformatics to identify gene sequences of potential interest from the many molecular biology databases now available. There is a continuing need to identify and characterise further genes and their related polypeptides/proteins, as targets for drug discovery.

### Summary of the Invention

The present invention relates to MAGI, in particular MAGI polypeptides and MAGI polynucleotides, recombinant materials and methods for their production. Such polypeptides and polynucleotides are of interest in relation to methods of treatment of certain diseases, including, but not limited to, neuropathies, spinal injury, neuronal degeneration, neuromuscular disorders, psychiatric disorders and developmental disorders, cancer, stroke and inflammatory disorders, hereinafter referred to as "diseases of the invention". In a further aspect, the invention relates to methods for identifying agonists and antagonists (*e.g.*, inhibitors) using the materials provided by the invention, and treating conditions associated with MAGI imbalance with the identified compounds In a still further aspect, the invention relates to diagnostic assays for detecting diseases associated with inappropriateMAGI activity or levels.

### Description of the Invention

In a first aspect, the present invention relates to MAGI polypeptides. Such polypeptides include

(a) an isolated polypeptide encoded by a polynucleotide comprising the sequence of SEQ ID NO:1 or SEQ ID NO:5;
(b) an isolated polypeptide comprising a polypeptide sequence having at least 95%, 96%, 97%, 98%, or 99% identity to the polypeptide sequence of SEQ ID NO:2 or SEQ ID NO:6;
(c) an isolated polypeptide comprising the polypeptide sequence of SEQ ID NO:2 or SEQ ID NO:6;
(d) an isolated polypeptide having at least 95%, 96%, 97%, 98%, or 99% identity to the polypeptide sequence of SEQ ID NO:2 or SEQ ID NO:6;
(e) the polypeptide sequence of SEQ ID NO:2 or SEQ ID NO:6; and
(f) an isolated polypeptide having or comprising a polypeptide sequence that has an Identity Index of 0.95, 0.96, 0.97, 0.98, or 0.99 compared to the polypeptide sequence of SEQ ID NO:2 or SEQ ID NO:6;
(g) fragments and variants of such polypeptides in (a) to (f).

Polypeptides of the present invention are believed to be members of the reticulon family of polypeptides. They are therefore of interest because members of this family have been shown to display prominent, but not exclusive, expression in cells of the nervous system. Expression of one isoform of these polypeptides, neuroendocrine specific protein C (NSP-C) (J. Hens et al. Cell Tissue Res. 292:229-237,1998), has been shown to correlate with neuronal differentiation. Alternative splicing of the genes of this family of polypeptides is known to generate differentially expressed isoforms with overlapping and distinct functions in different tissues (Geisler et al. Mamm. Genome 9:164-173, 1998). Amino acid similarity between members of this family of polypeptides with fragments of a high-molecular weight protein purified from bovine spinal cord (Spillmann et al. J. Biol. Chem. 273:15487-15493, 1998) indicates a potential role in axonal growth inhibition role for these proteins. Similarly, expression of neuroendocrine specific protein A (NSP-A) in specific cancerous cells (Senden et al. Histochem. Cell Biol. 108:155-165, 1997) may indicate a potential use of these polypeptides in the diagnosis and or treatment of cancers.

The detection of polynucleotides comprising portions of MAGI in human fetal brain and human adult spinal cord cDNA and an abundant >5kb mRNA isoform in human adult brain together with different transcripts potentially arising by alternative splicing in heart, lung, liver, kidney and skeletal muscle suggests that MAGI isoforms may similarly serve overlapping and distinct functions in these tissues. By analogy with the semaphorin family of neurite-modulatory polypeptides it might be postulated that these different isoforms would function in each of these tissues to control local innervation by distinct neuronal populations. Aberrant expression of specific isoforms within, for example, skeletal muscle would be predicted to alter motor and sensory neuron innervation in diseases such as amyotrophic lateral sclerosis (ALS).

The presence of characteristic signature polypeptides and highly hydrophobic regions in the polypeptide of the present invention suggests that its expression in regions of the nervous system and in tissues forming boundaries for growth may modulate growth and pathfinding both during development and following pathological or injurious processes.

Expression of the polypeptide of the invention, fragments thereof or alternatively spliced variants of the polypeptide on the surface of cells either naturally, as a secreted protein or following release by cellular damage may act on other cells either through specific receptors or pathologically through nonspecific interactions to modulate cell attachment, spreading, migration or growth. This inhibition might be expected to be either reversible or permanent possibly resulting in cell death..

The biological properties of the MAGI are hereinafter referred to as "biological activity of MAGI" or "MAGI activity". Preferably, a polypeptide of the present invention exhibits at least one biological activity of MAGI.

Polypeptides of the present invention also includes variants of the aforementioned polypeptides, including all allelic forms and splice variants. Such polypeptides vary from the reference polypeptide by insertions, deletions, and substitutions that may be conservative or non-conservative, or any combination thereof. Particularly preferred variants are those in which several, for instance from 50 to 30, from 30 to 20, from 20 to 10, from 10 to 5, from 5 to 3, from 3 to 2, from 2 to 1 or 1 amino acids are inserted, substituted, or deleted, in any combination.

Preferred fragments of polypeptides of the present invention include an isolated polypeptide comprising an amino acid sequence having at least 30, 50 or 100 contiguous amino acids from the amino acid sequence of SEQ ID NO: 2 or SEQ ID NO:6, or an isolated polypeptide comprising an amino acid sequence having at least 30, 50 or 100 contiguous amino acids truncated or deleted from the amino acid sequence of SEQ ID NO: 2 or SEQ ID NO:6. Preferred fragments are biologically active fragments that mediate the biological activity of MAGI, including those with a similar activity or an improved activity, or with a decreased undesirable activity. Also preferred are those fragments that are antigenic or immunogenic in an animal, especially in a human.

Fragments of the polypeptides of the invention may be employed for producing the corresponding full-length polypeptide by peptide synthesis; therefore, these variants may be employed as intermediates for producing the full-length polypeptides of the invention.Thepolypeptides of the present invention may be in the form of the "mature" protein or may be a part of a larger protein such as a precursor or a fusion protein. It is often advantageous to include an additional amino acid sequence that contains secretory or leader sequences, pro-sequences, sequences that aid in purification, for instance multiple histidine residues, or an additional sequence for stability during recombinant production.

Polypeptides of the present invention can be prepared in any suitable manner, for instance by isolation form naturally occuring sources, from genetically engineered host cells comprising expression systems (*vide infra*) or by chemical synthesis, using for instance automated peptide synthesisers, or a combination of such methods.. Means for preparing such polypeptides are well understood in the art.

In a further aspect, the present invention relates to MAGI polynucleotides. Such polynucleotides include:

(a) an isolated polynucleotide comprising a polynucleotide sequence having at least 95%, 96%, 97%, 98%, or 99% identity to the polynucleotide squence of SEQ ID NO:1 or SEQ ID NO:5;

(b) an isolated polynucleotide comprising the polynucleotide of SEQ ID NO: or SEQ ID NO:5;

(c) an isolated polynucleotide having at least 95%, 96%, 97%, 98%, or 99% identity to the polynucleotide of SEQ ID NO:1 or SEQ ID NO:5;

(d) the isolated polynucleotide of SEQ ID NO: or SEQ ID NO:5;

(e) an isolated polynucleotide comprising a polynucleotide sequence encoding a polypeptide sequence having at least 95%, 96%, 97%, 98%, or 99% identity to the polypeptide sequence of SEQ ID NO:2 or SEQ ID NO:6;

(f) an isolated polynucleotide comprising a polynucleotide sequence encoding the polypeptide of SEQ ID NO:2 or SEQ ID NO:6;

(g) an isolated polynucleotide having a polynucleotide sequence encoding a polypeptide sequence having at least 95%, 96%, 97%, 98%, or 99% identity to the polypeptide sequence of SEQ ID NO:2 or SEQ ID NO:6;

(h) an isolated polynucleotide encoding the polypeptide of SEQ ID NO:2 or SEQ ID NO:6;

(i) an isolated polynucleotide having or comprising a polynucleotide sequence that has an Identity Index of 0.95, 0.96, 0.97, 0.98, or 0.99 compared to the polynucleotide sequence of SEQ ID NO:1 or SEQ ID NO:5;

(j) an isolated polynucleotide having or comprising a polynucleotide sequence encoding a polypeptide sequence that has an Identity Index of 0.95, 0.96, 0.97, 0.98, or 0.99 compared to the polypeptide sequence of SEQ ID NO: 2 or SEQ ID NO:6; and

polynucleotides that are fragments and variants of the above mentioned polynucleotides or that are complementary to above mentioned polynucleotides, over the entire length thereof.

Preferred fragments of polynucleotides of the present invention include an isolated polynucleotide comprising an nucleotide sequence having at least 15, 30, 50 or 100 contiguous nucleotides from the sequence of SEQ ID NO: 1 or SEQ ID NO:5, or an isolated polynucleotide comprising an sequence having at least 30, 50 or 100 contiguous nucleotides truncated or deleted from the sequence of SEQ ID NO: 1 or SEQ ID NO:5.

Preferred variants of polynucleotides of the present invention include splice variants, allelic variants, and polymorphisms, including polynucleotides having one or more single nucleotide polymorphisms (SNPs).

Polynucleotides of the present invention also include polynucleotides encoding polypeptide variants that comprise the amino acid sequence of SEQ ID NO:2 or SEQ ID NO:6 and in which several, for instance from 50 to 30, from 30 to 20, from 20 to 10, from 10 to 5, from 5 to 3, from 3 to 2, from 2 to 1 or 1 amino acid residues are substituted, deleted or added, in any combination.

In a further aspect, the present invention provides polynucleotides that are RNA transcripts of the DNA sequences of the present invention. Accordingly, there is provided an RNA polynucleotide that:

(a) comprises an RNA transcript of the DNA sequence encoding the polypeptide of SEQ ID NO:2 or SEQ ID NO:6;
(b) is the RNA transcript of the DNA sequence encoding the polypeptide of SEQ ID NO:2 or SEQ ID NO:6;
(c) comprises an RNA transcript of the DNA sequence of SEQ ID NO:1 or SEQ ID NO:5; or
(d) is the RNA transcript of the DNA sequence of SEQ ID NO:1 or SEQ ID NO:5; and RNA polynucleotides that are complementary thereto.

The polynucleotide sequence of SEQ ID NO:1 shows homology with Human neuroendocrine-specific protein C (Roebroek et al., J. Biol. Chem. 268: 13439-13447,1993). The polynucleotide sequence of SEQ ID NO:1 is a cDNA sequence that encodes the polypeptide of SEQ ID NO:2. The polynucleotide sequence encoding the polypeptide of SEQ ID NO:2 may be identical to the polypeptide encoding sequence of SEQ ID NO:1 or it may be a sequence other than SEQ ID NO:1, which, as a result of the redundancy (degeneracy) of the genetic code, also encodes the polypeptide of SEQ ID NO:2. The polypeptide of the SEQ ID NO:2 is related to other proteins of the reticulon family, having homology and/or structural similarity with Human neuroendocrine-specific protein C (Roebroek et al., J. Biol. Chem. 268: 13439-13447,1993). The polynucleotide of SEQ ID NO:5 and the polypeptide encoded thereby, SEQ ID NO:6, is a variant of the polynucleotide of SEQ ID NO:1 and it's encoded polypeptide, SEQ ID NO:2.

Preferred polypeptides and polynucleotides of the present invention are expected to have, *inter alia*, similar biological functions/properties to their homologous polypeptides and polynucleotides. Furthermore, preferred polypeptides and polynucleotides of the present invention have at least one MAGI activity.

The present invention also relates to partial or other polynucleotide and polypeptide sequences which were first identified prior to the determination of the corresponding full length sequences of SEQ ID NO:1, SEQ ID NO:5, SEQ ID NO:2 and SEQ ID NO:6.

Accordingly, in a further aspect, the present invention provides for an isolated polynucleotide which:

(a) comprises a nucleotide sequence which has at least 95% identity, preferably at least 97-99% identity to SEQ ID NO:3 over the entire length of SEQ ID NO:3;
(b) has a nucleotide sequence which has at least 95% identity, preferably at least 97-99% identity, to SEQ ID NO: 3 over the entire length of SEQ ID NO:3;
(c) comprises the polynucleotide of SEQ ID NO:3; or
(d) has a nucleotide sequence encoding a polypeptide which has at least 95% identity, even more preferably at least 97-99% identity, to the amino acid sequence of SEQ ID NO:4, over the entire length of SEQ ID NO:4;
as well as the polynucleotide of SEQ ID NO:3.

The present invention further provides for a polypeptide which:

(a) comprises an amino acid sequence which has at least 95% identity, preferably at least 97-99% identity, to that of SEQ ID NO:4 over the entire length of SEQ ID NO:4;
(b) has an amino acid sequence which is at least 95% identity, preferably at least 97-99% identity, to the amino acid sequence of SEQ ID NO:4 over the entire length of SEQ ID NO:4;
(c) comprises the amino acid of SEQ ID NO:4; and
(d) is the polypeptide of SEQ ID NO:4;
as well as polypeptides encoded by a polynucleotide comprising the sequence contained in SEQ ID NO:3.

The nucleotide sequence of SEQ ID NO:3 and the peptide sequence encoded thereby are derived from EST (Expressed Sequence Tag) sequences. It is recognised by those skilled in the art that there will inevitably be some nucleotide sequence reading errors in EST sequences (see Adams, M.D. et al, Nature 377 (supp) 3, 1995). Accordingly, the nucleotide sequence of SEQ ID NO:3 and the peptide sequence encoded therefrom are therefore subject to the same inherent limitations in sequence accuracy. Furthermore, the peptide sequence encoded by SEQ ID NO:3 comprises a region of identity or close homology and/or close structural similarity (for example a conservative amino acid difference) with the closest homologous or structurally similar protein.

Polynucleotides of the present invention may be obtained using standard cloning and screening techniques from a cDNA library derived from mRNA in cells ofhuman fetal brain and spinal cord, (see for instance, Sambrook et al., Molecular Cloning: A Laboratory Manual, 2nd Ed., Cold Spring Harbor Laboratory Press, Cold Spring Harbor, N.Y. (1989)). Polynucleotides of the invention can also be obtained from natural sources such as genomic DNA libraries or can be synthesized using well known and commercially available techniques.

When polynucleotides of the present invention are used for the recombinant production of polypeptides of the present invention, the polynucleotide may include the coding sequence for the mature polypeptide, by itself, or the coding sequence for the mature polypeptide in reading frame with other coding sequences, such as those encoding a leader or secretory sequence, a pre-, or pro- or prepro- protein sequence, or other fusion peptide portions. For example, a marker sequence that facilitates purification of the fused polypeptide can be encoded. In certain preferred embodiments of this aspect of the invention, the marker sequence is a hexa-histidine peptide, as provided in the pQE vector (Qiagen, Inc.) and described in Gentz et al., Proc Natl Acad Sci USA (1989) 86:821-824, or is an HA tag. The polynucleotide may also contain noncoding 5' and 3' sequences, such as transcribed, non-translated sequences, splicing and polyadenylation signals, ribosome binding sites and sequences that stabilize mRNA.

Polynucleotides that are identical, or have sufficient identity to a polynucleotide sequence of SEQ ID NO:1 or SEQ ID NO:5, may be used as hybridization probes for cDNA and genomic DNA or as primers for a nucleic acid amplification reaction (for instance, PCR). Such probes and primers may be used to isolate full-length cDNAs and genomic clones encoding polypeptides of the present invention and to isolate cDNA and genomic clones of other genes (including genes encoding paralogs from human sources and orthologs and paralogs from species other than human) that have a high sequence similarity to SEQ ID NO:1 or SEQ ID NO:5, typically at least 95% identity. Preferred probes and primers will generally comprise at least 15 nucleotides, preferably, at least 30 nucleotides and may have at least 50, if not at least 100 nucleotides. Particularly preferred probes will have between 30 and 50 nucleotides. Particularly preferred primers will have between 20 and 25 nucleotides.

A polynucleotide encoding a polypeptide of the present invention, including homologs from species other than human, may be obtained by a process comprising the steps of screening a library under stringent hybridization conditions with a labeled probe having the sequence of SEQ ID NO: 1 or SEQ ID NO:5 or a fragment thereof, preferably of at least 15 nucleotides; and isolating full-length cDNA and genomic clones containing said polynucleotide sequence. Such hybridization techniques are well known to the skilled artisan. Preferred stringent hybridization conditions include overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA; followed by washing the filters in 0.1x SSC at about 65°C. Thus the present invention also includes isolated polynucleotides, preferably with a nucleotide sequence of at least 100, obtained by screening a library under stringent hybridization conditions with a labeled probe having the sequence of SEQ ID NO:1 or SEQ ID NO:5 or a fragment thereof, preferably of at least 15 nucleotides.

The skilled artisan will appreciate that, in many cases, an isolated cDNA sequence will be incomplete, in that the region coding for the polypeptide does not extend all the way through to the 5' terminus. This is a consequence of reverse transcriptase, an enzyme with inherently low "processivity" (a measure of the ability of the enzyme to remain attached to the template during the polymerisation reaction), failing to complete a DNA copy of the mRNA template during first strand cDNA synthesis.

There are several methods available and well known to those skilled in the art to obtain full-length cDNAs, or extend short cDNAs, for example those based on the method of Rapid Amplification of cDNA ends (RACE) (see, for example, Frohman et al., Proc Nat Acad Sci USA 85, 8998-9002, 1988). Recent modifications of the technique, exemplified by the Marathon (trade mark) technology (Clontech Laboratories Inc.) for example, have significantly simplified the search for longer cDNAs. In the Marathon (trade mark) technology, cDNAs have been prepared from mRNA extracted from a chosen tissue and an 'adaptor' sequence ligated onto each end. Nucleic acid amplification (PCR) is then carried out to amplify the "missing" 5' end of the cDNA using a combination of gene specific and adaptor specific oligonucleotide primers. The PCR reaction is then repeated using 'nested' primers, that is, primers designed to anneal within the amplified product (typically an adaptor specific primer that anneals further 3' in the adaptor sequence and a gene specific primer that anneals further 5' in the known gene sequence). The products of this reaction can then be analysed by DNA sequencing and a full-length cDNA constructed either by joining the product directly to the existing cDNA to give a complete sequence, or carrying out a separate full-length PCR using the new sequence information for the design of the 5' primer.

Recombinant polypeptides of the present invention may be prepared by processes well known in the art from genetically engineered host cells comprising expression systems. Accordingly, ina further aspect, the present invention relates to expression systems comprising a polynucleotide or polynucleotides of the present invention, to host cells which are genetically engineered with such expression sytems and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Polynucleotides may be introduced into host cells by methods described in many standard laboratory manuals, such as Davis et al, Basic Methods in Molecular Biology (1986) and Sambrook *et al.*(*ibid*). Preferred methods of introducing polynucleotides into host cells include, for instance, calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as*Streptococci, Staphylococci, E. coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used, for instance, chromosomal, episomal and virus-derived systems, *e.g.*, vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector that is able to maintain, propagate or express a polynucleotide to produce a polypeptide in a host may be used. The appropriate polynucleotide sequence may be inserted into an expression system by any of a variety of well-known and routine techniques, such as, for example, those set forth in Sambrook *et al.*, (*ibid*). Appropriate secretion signals may be incorporated into the desired polypeptide to allow secretion of the translated protein into the lumen of the endoplasmic reticulum, the periplasmic space or the extracellular environment. These signals may be endogenous to the polypeptide or they may be heterologous signals.

If a polypeptide of the present invention is to be expressed for use in screening assays, it is generally preferred that the polypeptide be produced at the surface of the cell. In this event, the cells may be harvested prior to use in the screening assay. If the polypeptide is secreted into the medium, the medium can be recovered in order to recover and purify the polypeptide. If produced intracellularly, the cells must first be lysed before the polypeptide is recovered.

Polypeptides of the present invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during intracellular synthesis, isolation and/or purification.

Polynucleotides of the present invention may be used as diagnostic reagents, through detecting mutations in the associated gene. Detection of a mutated form of the gene characterised by the polynucleotide of SEQ ID NO:1 or SEQ ID NO:5 in the cDNA or genomic sequence and which is associated with a dysfunction will provide a diagnostic tool that can add to, or define, a diagnosis of a disease, or susceptibility to a disease, which results from under-expression, over-expression or altered spatial or temporal expression of the gene. Individuals carrying mutations in the gene may be detected at the DNA level by a variety of techniques well known in the art.

Nucleic acids for diagnosis may be obtained from a subject's cells, such as from blood, urine, saliva, tissue biopsy or autopsy material. The genomic DNA may be used directly for detection or it may be amplified enzymatically by using PCR, preferably RT-PCR, or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeledMAGI nucleotide sequences. Perfectly matched sequences can be distinguished from mismatched duplexes by RNase digestion or by differences in melting temperatures. DNA sequence difference may also be detected by alterations in the electrophoretic mobility of DNA fragments in gels, with or without denaturing agents, or by direct DNA sequencing (see, for instance, Myers et al., Science (1985) 230:1242). Sequence changes at specific locations may also be revealed by nuclease protection assays, such as RNase and S1 protection or the chemical cleavage method (see Cotton et al., Proc Natl Acad Sci USA (1985) 85: 4397-4401).

An array of oligonucleotides probes comprisingMAGI polynucleotide sequence or fragments thereof can be constructed to conduct efficient screening of *e.g.*, genetic mutations. Such arrays are preferably high density arrays or grids. Array technology methods are well known and have general applicability and can be used to address a variety of questions in molecular genetics including gene expression, genetic linkage, and genetic variability, see, for example, M.Chee et al., Science, 274, 610-613 (1996) and other references cited therein.

Detection of abnormally decreased or increased levels of polypeptide or mRNA expression may also be used for diagnosing or determining susceptibility of a subjectto a disease of the invention. Decreased or increased expression can be measured at the RNA level using any of the methods well known in the art for the quantitation of polynucleotides, such as, for example, nucleic acid amplification, for instance PCR, RT-PCR, RNase protection, Northern blotting and other hybridization methods. Assay techniques that can be used to determine levels of a protein, such as a polypeptide of the present invention, in a sample derived from a host are well-known to those of skill in the art. Such assay methods include radioimmunoassays, competitive-binding assays, Western Blot analysis and ELISA assays.

Thus in another aspect, the present invention relates to a diagonostic kit comprising:

(a) a polynucleotide of the present invention, preferably the nucleotide sequence of SEQ ID NO: I or SEQ ID NO: 5, or a fragment or an RNA transcript thereof;
(b) a nucleotide sequence complementary to that of (a);
(c) a polypeptide of the present invention, preferably the polypeptide of SEQ ID NO:2 or SEQ ID NO:6 or a fragment thereof; or
(d) an antibody to a polypeptide of the present invention, preferably to the polypeptide of SEQ ID NO:2 or SEQ ID NO:6.

It will be appreciated that in any such kit, (a), (b), (c) or (d) may comprise a substantial component. Such a kit will be of use in diagnosing a disease or susceptibility to a disease, particularly diseases of the invention, amongst others.

The polynucleotide sequences of the present invention are valuable for chromosome localisation studies. The sequence is specifically targeted to, and can hybridize with, a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found in, for example, V. McKusick, Mendelian Inheritance in Man (available on-line through Johns Hopkins University Welch Medical Library). The relationship between genes and diseases that have been mapped to the same chromosomal region are then identified through linkage analysis (co-inheritance of physically adjacent genes). Precise human chromosomal localisations for a genomic sequence (gene fragment etc.) can be determined using Radiation Hybrid (RH) Mapping (Walter, M. Spillett, D., Thomas, P., Weissenbach, J., and Goodfellow, P., (1994) A method for constructing radiation hybrid maps of whole genomes, Nature Genetics 7, 22-28). A number of RH panels are available from Research Genetics (Huntsville, AL, USA) e.g. the GeneBridge4 RH panel (Hum Mol Genet 1996 Mar;5(3):339-46 A radiation hybrid map of the human genome. Gyapay G, Schmitt K, Fizames C, Jones H, Vega-Czarny N, Spillett D, Muselet D, Prud'Homme JF, Dib C, Auffray C, Morissette J, Weissenbach J, Goodfellow PN). To determine the chromosomal location of a gene using this panel, 93 PCRs are performed using primers designed from the gene of interest on RH DNAs. Each of these DNAs contains random human genomic fragments maintained in a hamster background (human / hamster hybrid cell lines). These PCRs result in 93 scores indicating the presence or absence of the PCR product of the gene of interest. These scores are compared with scores created using PCR products from genomic sequences of known location. This comparison is conducted at http://www.genome.wi.mit.edu/. The gene of the present invention maps to human chromosome2p21.

The polynucleotide sequences of the present invention are also valuable tools for tissue expression studies. Such studies allow the determination of expression patterns of polynucleotides of the present invention which may give an indication as to the expression patterns of the encoded polypeptides in tissues, by detecting the mRNAs that encode them. The techniques used are well known in the art and include in situ hydridisation techniques to clones arrayed on a grid, such as cDNA microarray hybridisation (Schena et al, Science, 270, 467-470, 1995 and Shalon et al, Genome Res, 6, 639-645, 1996) and nucleotide amplification techniques such as PCR. A preferred method uses the TAQMAN (Trade mark) technology available from Perkin Elmer. Results from these studies can provide an indication of the normal function of the polypeptide in the organism. In addition, comparative studies of the normal expression pattern of mRNAs with that of mRNAs encoded by an alternative form of the same gene (for example, one having an alteration in polypeptide coding potential or a regulatory mutation) can provide valuable insights into the role of the polypeptides of the present invention, or that of inappropriate expression thereof in disease. Such inappropriate expression may be of a temporal, spatial or simply quantitative nature The polypeptides of the present invention are expressed in brain, heart, liver, skeletal muscle, pancreas and kidney, based on Northern blot data provided in figure 1.

A further aspect of the present invention relates to antibodies. The polypeptides of the invention or their fragments, or cells expressing them, can be used as immunogens to produce antibodies that are immunospecific for polypeptides of the present invention. The term "immunospecific" means that the antibodies have substantially greater affinity for the polypeptides of the invention than their affinity for other related polypeptides in the prior art.

Antibodies generated against polypeptides of the present invention may be obtained by administering the polypeptides or epitope-bearing fragments, or cells to an animal, preferably a non-human animal, using routine protocols. For preparation of monoclonal antibodies, any technique which provides antibodies produced by continuous cell line cultures can be used. Examples include the hybridoma technique (Kohler, G. and Milstein, C., Nature (1975) 256: 495-497), the trioma technique, the human B-cell hybridoma technique (Kozbor et al., Immunology Today (1983) 4:72) and the EBV-hybridoma technique (Cole et al., Monoclonal Antibodies and Cancer Therapy, 77-96, Alan R. Liss, Inc., 1985).

Techniques for the production of single chain antibodies, such as those described in U.S. Patent No. 4,946,778, can also be adapted to produce single chain antibodies to polypeptides of this invention. Also, transgenic mice, or other organisms, including other mammals, may be used to express humanized antibodies.

The above-described antibodies may be employed to isolate or to identify clones expressing the polypeptide or to purify the polypeptides by affinity chromatography. Antibodies against polypeptides of the present invention may also be employed to treat diseases of the invention, amongst others.

Polypeptides and polynucleotides of the present invention may also be used as vaccines. Accordingly, in a further aspect, the present invention relates to a method for inducing an immunological response in a mammal that comprises inoculating the mammal with a polypeptide of the present invention, adequate to produce antibody and/or T cell immune response, including, for example, cytokine-producing T cells or cytotoxic T cells, to protect said animal from disease whether that disease is already established within the individual or not. An immunological response in a mammal may also be induced by a method comprises delivering a polypeptide of the present invention *via* a vector directing expression of the polynucleotide and coding for the polypeptide *in vivo* in order to induce such an immunological response to produce antibody to protect said animal from diseases of the invention. One way of administering the vector is by accelerating it into the desired cells as a coating on particles or otherwise. Such nucleic acid vector may comprise DNA, RNA, a modified nucleic acid, or a DNA/RNA hybrid. For use a vaccine, a polypeptide or a nucleic acid vector will be normally provided as a vaccine formulation (composition). The formulation may further comprise a suitable carrier. Since a polypeptide may be broken down in the stomach, it is preferably administered parenterally (for instance, subcutaneous, intramuscular, intravenous, or intradermal injection). Formulations suitable for parenteral administration include aqueous and non-aqueous sterile injection solutions that may contain anti-oxidants, buffers, bacteriostats and solutes that render the formulation instonic with the blood of the recipient; and aqueous and non-aqueous sterile suspensions that may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

Polypeptides of the present invention have one or more biological functions that are of relevance in one or more disease states, in particular the diseases of the invention hereinbefore mentioned. It is therefore useful to to identify compounds that stimulate or inhibit the function or level of the polypeptide. Accordingly, in a further aspect, the present invention provides for a method of screening compounds to identify those that stimulate or inhibit the function or level of the polypeptide. Such methods identify agonists or antagonists that may be employed for therapeutic and prophylactic purposes for such diseases of the invention as hereinbefore mentioned. Compounds may be identified from a variety of sources, for example, cells, cell-free preparations, chemical libraries, collections of chemical compounds, and natural product mixtures. Such agonists or antagonists so-identified may be natural or modified substrates, ligands, receptors, enzymes, etc., as the case may be, of the polypeptide; a structural or functional mimetic thereof (see Coligan et al., Current Protocols in Immunology 1(2):Chapter 5 (1991)) or a small molecule.

The screening method may simply measure the binding of a candidate compound to the polypeptide, or to cells or membranes bearing the polypeptide, or a fusion protein thereof, by means of a label directly or indirectly associated with the candidate compound. Alternatively, the screening method may involve measuring or detecting (qualitatively or quantitatively) the competitive binding of a candidate compound to the polypeptide against a labeled competitor (*e.g.* agonist or antagonist). Further, these screening methods may test whether the candidate compound results in a signal generated by activation or inhibition of the polypeptide, using detection systems appropriate to the cells bearing the polypeptide. Inhibitors of activation are generally assayed in the presence of a known agonist and the effect on activation by the agonist by the presence of the candidate compound is observed. Further, the screening methods may simply comprise the steps of mixing a candidate compound with a solution containing a polypeptide of the present invention, to form a mixture, measuring a MAGI activity in the mixture, and comparing the MAGI activity of the mixture to a control mixture which contains no candidate compound.

Polypeptides of the present invention may be employed in conventional low capacity screening methods and also in high-throughput screening (HTS) formats. Such HTS formats include not only the well-established use of 96- and, more recently, 384-well micotiter plates but also emerging methods such as the nanowell method described by Schullek et al, Anal Biochem., 246, 20-29, (1997).

Fusion proteins, such as those made from Fc portion and MAGI polypeptide, as hereinbefore described, can also be used for high-throughput screening assays to identify antagonists for the polypeptide of the present invention (see D. Bennett et al., J Mol Recognition, 8:52-58 (1995); and K. Johanson et al., J Biol Chem, 270(16):9459-9471 (1995)).

The polynucleotides, polypeptides and antibodies to the polypeptide of the present invention may also be used to configure screening methods for detecting the effect of added compounds on the production of mRNA and polypeptide in cells. For example, an ELISA assay may be constructed for measuring secreted or cell associated levels of polypeptide using monoclonal and polyclonal antibodies by standard methods known in the art. This can be used to discover agents that may inhibit or enhance the production of polypeptide (also called antagonist or agonist, respectively) from suitably manipulated cells or tissues.

A polypeptide of the present invention may be used to identify membrane bound or soluble receptors, if any, through standard receptor binding techniques known in the art. These include, but are not limited to, ligand binding and crosslinking assays in which the polypeptide islabeled with a radioactive isotope (for instance, $^{125}I$), chemically modified (for instance, biotinylated), or fused to a peptide sequence suitable for detection or purification, and incubated with a source of the putative receptor (cells, cell membranes, cell supernatants, tissue extracts, bodily fluids). Other methods include biophysical techniques such as surface plasmon resonance and spectroscopy. These screening methods may also be used to identify agonists and antagonists of the polypeptide that compete with the binding of the polypeptide to its receptors, if any. Standard methods for conducting such assays are well understood in the art.

Examples of antagonists of polypeptides of the present invention include antibodies or, in some cases, oligonucleotides or proteins that are closely related to the ligands, substrates, receptors, enzymes, etc., as the case may be, of the polypeptide, *e.g.*, a fragment of the ligands, substrates, receptors, enzymes, etc.; or a small molecule that bind to the polypeptide of the present invention but do not elicit a response, so that the activity of the polypeptide is prevented.

Screening methods may also involve the use of transgenic technology and MAGI gene. The art of constructing transgenic animals is well established. For example, the MAGI gene may be introduced through microinjection into the male pronucleus of fertilized oocytes, retroviral transfer into pre- or post-implantation embryos, or injection of genetically modified, such as by electroporation, embryonic stem cells into host blastocysts. Particularly useful transgenic animals are so-called "knock-in" animals in which an animal gene is replaced by the human equivalent within the genome of that animal. Knock-in transgenic animals are useful in the drug discovery process, for target validation, where the compound is specific for the human target. Other useful transgenic animals are so-called "knock-out" animals in which the expression of the animal ortholog of a polypeptide of the present invention and encoded by an endogenous DNA sequence in a cell is partially or completely annulled. The gene knock-out may be targeted to specific cells or tissues, may occur only in certain cells or tissues as a consequence of the limitations of the technology, or may occur in all, or substantially all, cells in the animal. Transgenic animal technology also offers a whole animal expression-cloning system in which introduced genes are expressed to give large amounts of polypeptides of the present invention

Screening kits for use in the above described methods form a further aspect of the present invention. Such screening kits comprise:

(a) a polypeptide of the present invention;
(b) a recombinant cell expressing a polypeptide of the present invention;
(c) a cell membrane expressing a polypeptide of the present invention; or
(d) an antibody to a polypeptide of the present invention;
which polypeptide is preferably that of SEQ ID NO:2 or SEQ ID NO:6.

It will be appreciated that in any such kit, (a), (b), (c) or (d) may comprise a substantial component.

## Glossary

The following definitions are provided to facilitate understanding of certain terms used frequently hereinbefore.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from its natural state, *i.e.*, if it occurs in nature, it has been changed or removed from its original environment, or both. For example, a polynucleotide or a polypeptide naturally present in a living organism is not "isolated," but the same polynucleotide or polypeptide separated from the coexisting materials of its natural state is "isolated", as the term is employed herein. Moreover, a polynucleotide or polypeptide that is introduced into an organism by transformation, genetic manipulation or by any other recombinant method is "isolated" even if it is still present in said organism, which organism may be living or non-living.

"Polynucleotide" generally refers to any polyribonucleotide (RNA) or polydeoxribonucleotide (DNA), which may be unmodified or modified RNA or DNA. "Polynucleotides" include, without limitation, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term "polynucleotide" also includes DNAs or RNAs containing one or more modified bases and DNAs or RNAs with backbones modified for stability or for other reasons. "Modified" bases include, for example, tritylated bases and unusual bases such as inosine. A variety of modifications may be made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any polypeptide comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques that are well known in the art. Such modifications are well described in basic texts and in more detailed monographs, as well as in a voluminous research literature. Modifications may occur anywhere in a polypeptide, including the peptide backbone, the amino acid side-chains and the amino or carboxyl termini. It will be appreciated that the same type of modification may be present to the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, biotinylation, covalent attachment of flavin, covalent attachment of a heme moiety, covalent attachment of a nucleotide or-nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination (see, for instance, Proteins - Structure and Molecular Properties, 2nd Ed., T. E. Creighton, W. H. Freeman and Company, New York, 1993; Wold, F., Post-translational Protein Modifications: Perspectives and Prospects, 1-12, in Post-translational Covalent Modification of Proteins, B. C. Johnson, Ed., Academic Press, New York, 1983; Seifter et al., "Analysis for protein modifications and nonprotein cofactors", Meth Enzymol, 182, 626-646, 1990, and Rattan et al., "Protein Synthesis: Post-translational Modifications and Aging", Ann NY Acad Sci, 663, 48-62, 1992). "Fragment" of a polypeptide sequence refers to a polypeptide sequence that is shorter than the reference sequence but that retains essentially the same biological function or activity as the reference polypeptide. "Fragment" of a polynucleotide sequence refers to a polynucloetide sequence that is shorter than the reference sequence of SEQ ID NO:1 or SEQ ID NO:5.

"Variant" refers to a polynucleotide or polypeptide that differs from a reference polynucleotide or polypeptide, but retains the essential properties thereof. A typical variant of a polynucleotide differs in nucleotide sequence from the reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from the reference polypeptide. Generally, alterations are limited so that the sequences of the reference polypeptide and the variant are closely similar overall and, in many regions, identical. A variant and reference polypeptide may differ in amino acid sequence by one or more substitutions, insertions, deletions in any combination. A substituted or inserted amino acid residue may or may not be one encoded by the genetic code. Typical conservative substitutions include Gly, Ala; Val, Ile, Leu; Asp, Glu; Asn, Gln; Ser, Thr; Lys, Arg; and Phe and Tyr. A variant of a polynucleotide or polypeptide may be naturally occurring such as an allele, or it may be a variant that is not known to occur naturally. Non-naturally occurring variants of polynucleotides and polypeptides may be made by mutagenesis techniques or by direct synthesis. Also included as variants are polypeptides having one or more post-translational modifications, for instance glycosylation, phosphorylation, methylation, ADP ribosylation and the like. Embodiments include methylation of the N-terminal amino acid, phosphorylations of serines and threonines and modification of C-terminal glycines.

"Allele" refers to one of two or more alternative forms of a gene occuring at a given locus in the genome.

"Polymorphism" refers to a variation in nucleotide sequence (and encoded polypeptide sequence, if relevant) at a given position in the genome within a population.

"Single Nucleotide Polymorphism" (SNP) refers to the occurence of nucleotide variability at a single nucleotide position in the genome, within a population. An SNP may occur within a gene or within intergenic regions of the genome. SNPs can be assayed using Allele Specific Amplification (ASA). For the process at least 3 primers are required. A common primer is used in reverse complement to the polymorphism being assayed. This common primer can be between 50 and 1500 bps from the polymorphic base. The other two (or more) primers are identical to each other except that the final 3' base wobbles to match one of the two (or more) alleles that make up the polymorphism. Two (or more) PCR reactions are then conducted on sample DNA, each using the common primer and one of the Allele Specific Primers.

"Splice Variant" as used herein refers to cDNA molecules produced from RNA molecules initially transcribed from the same genomic DNA sequence but which have undergone alternative RNA splicing. Alternative RNA splicing occurs when a primary RNA transcript undergoes splicing, generally for the removal of introns, which results in the production of more than one mRNA molecule each of that may encode different amino acid sequences. The term splice variant also refers to the proteins encoded by the above cDNA molecules.

"Identity" reflects a relationship between two or more polypeptide sequences or two or more polynucleotide sequences, determined by comparing the sequences. In general, identity refers to an exact nucleotide to nucleotide or amino acid to amino acid correspondence of the two polynucleotide or two polypeptide sequences, respectively, over the length of the sequences being compared.

"% Identity" - For sequences where there is not an exact correspondence, a "% identity" may be determined. In general, the two sequences to be compared are aligned to give a maximum correlation between the sequences. This may include inserting "gaps" in either one or both sequences, to enhance the degree of alignment. A % identity may be determined over the whole length of each of the sequences being compared (so-called global alignment), that is particularly suitable for sequences of the same or very similar length, or over shorter, defined lengths (so-called local alignment), that is more suitable for sequences of unequal length.

"Similarity" is a further, more sophisticated measure of the relationship between two polypeptide sequences. In general, "similarity" means a comparison between the amino acids of two polypeptide chains, on a residue by residue basis, taking into account not only exact correspondences between a between pairs of residues, one from each of the sequences being compared (as for identity) but also, where there is not an exact correspondence, whether, on an evolutionary basis, one residue is a likely substitute for the other. This likelihood has an associated "score" from which the "% similarity" of the two sequences can then be determined.

Methods for comparing the identity and similarity of two or more sequences are well known in the art. Thus for instance, programs available in the Wisconsin Sequence Analysis Package, version 9.1 (Devereux J et al, Nucleic Acids Res, 12, 387-395, 1984, available from Genetics Computer Group, Madison, Wisconsin, USA), for example the programs BESTFIT and GAP, may be used to determine the % identity between two polynucleotides and the % identity and the % similarity between two polypeptide sequences. BESTFIT uses the "local homology" algorithm of Smith and Waterman (J Mol Biol, 147,195-197, 1981, Advances in Applied Mathematics, 2, 482-489, 1981) and finds the best single region of similarity between two sequences. BESTFIT is more suited to comparing two polynucleotide or two polypeptide sequences that are dissimilar in length, the program assuming that the shorter sequence represents a portion of the longer. In comparison, GAP aligns two sequences, finding a "maximum similarity", according to the algorithm of Neddleman and Wunsch (J Mol Biol, 48, 443-453, 1970). GAP is more suited to comparing sequences that are approximately the same length and an alignment is expected over the entire length. Preferably, the parameters "Gap Weight" and "Length Weight" used in each program are 50 and 3, for polynucleotide sequences and 12 and 4 for polypeptide sequences, respectively. Preferably, % identities and similarities are determined when the two sequences being compared are optimally aligned.

Other programs for determining identity and/or similarity between sequences are also known in the art, for instance the BLAST family of programs (Altschul S F et al, J Mol Biol, 215, 403-410, 1990, Altschul S F et al, Nucleic Acids Res., 25:389-3402, 1997, available from the National Center for Biotechnology Information (NCBI), Bethesda, Maryland, USA and accessible through the home page of the NCBI at www.ncbi.nlm.nih.gov) and FASTA (Pearson W R, Methods in Enzymology, 183, 63-99, 1990; Pearson W R and Lipman D J, Proc Nat Acad Sci USA, 85, 2444-2448,1988, available as part of the Wisconsin Sequence Analysis Package).

Preferably, the BLOSUM62 amino acid substitution matrix (Henikoff S and Henikoff J G, Proc. Nat. Acad Sci. USA, 89, 10915-10919, 1992) is used in polypeptide sequence comparisons including where nucleotide sequences are first translated into amino acid sequences before comparison.

Preferably, the program BESTFIT is used to determine the % identity of a query polynucleotide or a polypeptide sequence with respect to a reference polynucleotide or a polypeptide sequence, the query and the reference sequence being optimally aligned and the parameters of the program set at the default value, as hereinbefore described.

"Identity Index" is a measure of sequence relatedness which may be used to compare a candidate sequence (polynucleotide or polypeptide) and a reference sequence. Thus, for instance, a candidate polynucleotide sequence having, for example, an Identity Index of 0.95 compared to a reference polynucleotide sequence is identical to the reference sequence except that the candidate polynucleotide sequence may include on average up to five differences per each 100 nucleotides of the reference sequence. Such differences are selected from the group consisting of at least one nucleotide deletion, substitution, including transition and transversion, or insertion. These differences may occur at the 5' or 3' terminal positions of the reference polynucleotide sequence or anywhere between these terminal positions, interspersed either individually among the nucleotides in the reference sequence or in one or more contiguous groups within the reference sequence. In other words, to obtain a polynucleotide sequence having an Identity Index of 0.95 compared to a reference polynucleotide sequence, an average of up to 5 in every 100 of the nucleotides of the in the reference sequence may be deleted, substituted or inserted, or any combination thereof, as hereinbefore described. The same applies *mutatis mutandis* for other values of the Identity Index, for instance 0.96, 0.97, 0.98 and 0.99.

Similarly, for a polypeptide, a candidate polypeptide sequence having, for example, an Identity Index of 0.95 compared to a reference polypeptide sequence is identical to the reference sequence except that the polypeptide sequence may include an average of up to five differences per each 100 amino acids of the reference sequence. Such differences are selected from the group consisting of at least one amino acid deletion, substitution, including conservative and non-conservative substitution, or insertion. These differences may occur at the amino- or carboxy-terminal positions of the reference polypeptide sequence or anywhere between these terminal positions, interspersed either individually among the amino acids in the reference sequence or in one or more contiguous groups within the reference sequence. In other words, to obtain a polypeptide sequence having an Identity Index of 0.95 compared to a reference polypeptide sequence, an average of up to 5 in every 100 of the amino acids in the reference sequence may be deleted, substituted or inserted, or any combination thereof, as hereinbefore described. The same applies *mutatis mutandis* for other values of the Identity Index, for instance 0.96, 0.97, 0.98 and 0.99.

The relationship between the number of nucleotide or amino acid differences and the Identity Index may be expressed in the following equation:

$$\mathbf{n_a \leq x_a - (x_a \bullet I)},$$

in which:

$\mathbf{n_a}$ is the number of nucleotide or amino acid differences,
$\mathbf{x_a}$ is the total number of nucleotides or amino acids in SEQ ID NO:1 or SEQ ID NO:5 or SEQ ID NO:2 or SEQ ID NO:6, respectively,
**I** is the Identity Index,

- is the symbol for the multiplication operator, and
  in which any non-integer product of $\mathbf{x_a}$ and **I** is rounded down to the nearest integer prior to subtracting it from $\mathbf{x_a}$.

"Homolog" is a generic term used in the art to indicate a polynucleotide or polypeptide sequence possessing a high degree of sequence relatedness to a reference sequence. Such relatedness may be quantified by determining the degree of identity and/or similarity between the two sequences as hereinbefore defined. Falling within this generic term are the terms "ortholog", and "paralog". "Ortholog" refers to a polynucleotide or polypeptide that is the functional equivalent of the polynucleotide or polypeptide in another species. "Paralog" refers to a polynucleotideor polypeptide that within the same species which is functionally similar.

"Fusion protein" refers to a protein encoded by two, often unrelated, fused genes or fragments thereof. In one example, EP-A-0 464 *** discloses fusion proteins comprising various portions of constant region of immunoglobulin molecules together with another human protein or part thereof. In many cases, employing an immunoglobulin Fc region as a part of a fusion protein is advantageous for use in therapy and diagnosis resulting in, for example, improved pharmacokinetic properties [see, *e.g.*, EP-A 0232 262]. On the other hand, for some uses it would be desirable to be able to delete the Fc part after the fusion protein has been expressed, detected and purified.

**Example - Tissue distribution of human MAGI**

A radioactively labelled DNA probe corresponding to the C-terminal 2kb of the MAGI open reading frame was hybridised with a Clontech MTN-1 filter containing equivalent loadings of mRNA from each of the indicated tissues and then washed to high stringency to only detect MAGI transcripts. The results are shown in figure 1. At least three bands are visible (>5kb, 2.4kb and <2kb). The largest transcript is present in adult brain and to a lesser degree in heart and skeletal muscle. The middle transcript is present at essentially similar levels in all tissues while the shortest transcripts is more specifically expressed, most abundantly in skeletal muscle, brain and kidney with low levels detectable in the pancreas.

Figure 1 Depicts a Northern blot showing tissue distribution of human MAGI. Lane 1 contains human adult heart RNA, lane 2 contains human adult brain RNA, lane 3 contains human placental RNA, lane 4 contains human adult lung RNA, lane 5 contains human adult liver RNA , lane 6 contains human adult skeletal muscle RNA, lane 7 contains human adult kidney RNA and lane 8 contains human adult pancreas RNA.

All publications and references, including but not limited to patents and patent applications, cited in this specification are herein incorporated by reference in their entirety as if each individual publication or reference were specifically and individually indicated to be incorporated by reference herein as being fully set forth. Any patent application to which this application claims priority is also incorporated by reference herein in its entirety in the manner described above for publications and references.

## SEQUENCE INFORMATION

## SEQ ID NO:1

ATGGAAGACCTGGACCAGTCTCCTCTGGTCTCGTCCTCGGACAGCCCACCCCGGCCGCAG
CCCGCGTTCAAGTACCAGTTCGTGAGGGAGCCCGAGGACGAGGAGGAAGAAGAGGAGGAG
GAAGAGGAGGACGAGGACGAAGACCTGGAGGAGCTGGAGGTGCTGGAGAGGAAGCCCGCC
GCCGGGCTGTCCGCGGCCCCAGTGCCCACCGCCCCTGCCGCCGGCGCGCCCCTGATGGAC
TTCGGAAATGACTTCGTGCCGCCGGCGCCCCGGGGACCCCTGCCGGCCGCTCCCCCCGTC
GCCCCGGAGCGGCAGCCGTCTTGGGACCCGAGCCCGGTGTCGTCGACCGTGCCCGCGCCA
TCCCCGCTGTCTGCTGCCGCAGTCTCGCCCTCCAAGCTCCCTGAGGACGACGAGCCTCCG
GCCCGGCCTCCCCCTCCTCCCCCGGCCAGCGTGAGCCCCCAGGCAGAGCCCGTGTGGACC
CCGCCAGCCCCGGCTCCCGCCGCGCCCCCCTCCACCCCGGCCGCGCCCAAGCGCAGGGGC
TCCTCGGGCTCAGTGGATGAGACCCTTTTTGCTCTTCCTGCTGCATCTGAGCCTGTGATA
CGCTCCTCTGCAGAAAATATGGACTTGAAGGAGCAGCCAGGTAACACTATTTCGGCTGGT
CAAGAGGATTTCCCATCTGTCCTGCTTGAAACTGCTGCTTCTCTTCCTTCTCTGTCTCCT
CTCTCAGCCGCTTCTTTCAAAGAACATGAATACCTTGGTAATTTGTCAACAGTATTACCC
ACTGAAGGAACACTTCAAGAAAATGTCAGTGAAGCTTCTAAAGAGGTCTCAGAGAAGGCA
AAAACTCTACTCATAGATAGAGATTTAACAGAGTTTTCAGAATTAGAATACTCAGAAATG
GGATCATCGTTCAGTGTCTCTCCAAAAGCAGAATCTGCCGTAATAGTAGCAAATCCTAGG
GAAGAAATAATCGTGAAAAATAAAGATGAAGAAGAGAAGTTAGTTAGTAATAACATCCTT
CATAATCAACAAGAGTTACCTACAGCTCTTACTAAATTGGTTAAAGAGGATGAAGTTGTG
TCTTCAGAAAAAGCAAAAGACAGTTTTAATGAAAAGAGAGTTGCAGTGGAAGCTCCTATG
AGGGAGGAATATGCAGACTTCAAACCATTTGAGCGAGTATGGGAAGTGAAAGATAGTAAG
GAAGATAGTGATATGTTGGCTGCTGGAGGTAAAATCGAGAGCAACTTGGAAAGTAAAGTG
GATAAAAAATGTTTTGCAGATAGCCTTGAGCAAACTAATCACGAAAAAGATAGTGAGAGT
AGTAATGATGATACTTCTTTCCCCAGTACGCCAGAAGGTATAAAGGATCGTCCAGGAGCA
TATATCACATGTGCTCCCTTTAACCCAGCAGCAACTGAGAGCATTGCAACAAACATTTTT
CCTTTGTTAGGAGATCCTACTTCAGAAAATAAGACCGATGAAAAAAAAATAGAAGAAAAG
AAGGCCCAAATAGTAACAGAGAAGAATACTAGCACCAAAACATCAAACCCTTTTCTTGTA
GCAGCACAGGATTCTGAGACAGATTATGTCACAACAGATAATTTAACAAAGGTGACTGAG
GAAGTCGTGGCAAACATGCCTGAAGGCCTGACTCCAGATTTAGTACAGGAAGCATGTGAA
AGTGAATTGAATGAAGTTACTGGTACAAAGATTGCTTATGAAACAAAAATGGACTTGGTT
CAAACATCAGAAGTTATGCAAGAGTCACTCTATCCTGCAGCACAGCTTTGCCCATCATTT
GAAGAGTCAGAAGCTACTCCTTCACCAGTTTTGCCTGACATTGTTATGGAAGCACCATTG
AATTCTGCAGTTCCTAGTGCTGGTGCTTCCGTGATACAGCCCAGCTCATCACCATTAGAA
GCTTCTTCAGTTAATTATGAAAGCATAAAACATGAGCCTGAAAACCCCCCACCATATGAA
GAGGCCATGAGTGTATCACTAAAAAAAGTATCAGGAATAAAGGAAGAAATTAAAGAGCCT
GAAAATATTAATGCAGCTCTTCAAGAAACAGAAGCTCCTTATATATCTATTGCATGTGAT
TTAATTAAAGAAACAAAGCTTTCTGCTGAACCAGCTCCGGATTTCTCTGATTATTCAGAA
ATGGCAAAAGTTGAACAGCCAGTGCCTGATCATTCTGAGCTAGTTGAAGATTCCTCACCT
GATTCTGAACCAGTTGACTTATTTAGTGATGATTCAATACCTGACGTTCCACAAAAACAA
GATGAAACTGTGATGCTTGTGAAAGAAAGTCTCACTGAGACTTCATTTGAGTCAATGATA

GAATATGAAAATAAGGAAAAACTCAGTGCTTTGCCACCTGAGGGAGGAAAGCCATATTTG
GAATCTTTTAAGCTCAGTTTAGATAACACAAAAGATACCCTGTTACCTGATGAAGTTTCA
ACATTGAGCAAAAAGGAGAAAATTCCTTTGCAGATGGAGGAGCTCAGTACTGCAGTTTAT
TCAAATGATGACTTATTTATTTCTAAGGAAGCACAGATAAGAGAAACTGAAACGTTTTCA
GATTCATCTCCAATTGAAATTATAGATGAGTTCCCTACATTGATCAGTTCTAAAACTGAT
TCATTTTCTAAATTAGCCAGGGAATATACTGACCTAGAAGTATCCCACAAAAGTGAAATT
GCTAATGCCCCGGATGGAGCTGGGTCATTGCCTTGCACAGAATTGCCCCATGACCTTTCT
TTGAAGAACATACAACCCAAAGTTGAAGAGAAAATCAGTTTCTCAGATGACTTTTCTAAA
AATGGGTCTGCTACATCAAAGGTGCTCTTATTGCCTCCAGATGTTTCTGCTTTGGCCACT
CAAGCAGAGATAGAGAGCATAGTTAAACCCAAAGTTCTTGTGAAAGAAGCTGAGAAAAAA
CTTCCTTCCGATACAGAAAAAGAGGACAGATCACCATCTGCTATATTTTCAGCAGAGCTG
AGTAAAACTTCAGTTGTTGACCTCCTGTACTGGAGAGACATTAAGAAGACTGGAGTGGTG
TTTGGTGCCAGCCTATTCCTGCTGCTTTCATTGACAGTATTCAGCATTGTGAGCGTAACA
GCCTACATTGCCTTGGCCCTGCTCTCTGTGACCATCAGCTTTAGGATATACAAGGGTGTG
ATCCAAGCTATCCAGAAATCAGATGAAGGCCACCCATTCAGGGCATATCTGGAATCTGAA
GTTGCTATATCTGAGGAGTTGGTTCAGAAGTACAGTAATTCTGCTCTTGGTCATGTGAAC
TGCACGATAAAGGAACTCAGGCGCCTCTTCTTAGTTGATGATTTAGTTGATTCTCTGAAG
TTTGCAGTGTTGATGTGGGTATTTACCTATGTTGGTGCCTTGTTTAATGGTCTGACACTA
CTGATTTTGGCTCTCATTTCACTCTTCAGTGTTCCTGTTATTTATGAACGGCATCAGGCG
CAGATAGATCATTATCTAGGACTTGCAAATAAGAATGTTAAAGATGCTATGGCTAAAATC
CAAGCAAAAATCCCTGGATTGAAGCGCAAAGCTGAATGA

**SEQ ID NO:2**

MEDLDQSPLVSSSDSPPRPQPAFKYQFVREPEDEEEEEEEEEEDEDEDLEELEVLERKPA
AGLSAAPVPTAPAAGAPLMDFGNDFVPPAPRGPLPAAPPVAPERQPSWDPSPVSSTVPAP
SPLSAAAVSPSKLPEDDEPPARPPPPPPASVSPQAEPVWTPPAPAPAAPPSTPAAPKRRG
SSGSVDETLFALPAASEPVIRSSAENMDLKEQPGNTISAGQEDFPSVLLETAASLPSLSP
LSAASFKEHEYLGNLSTVLPTEGTLQENVSEASKEVSEKAKTLLIDRDLTEFSELEYSEM
GSSFSVSPKAESAVIVANPREEIIVKNKDEEEKLVSNNILHNQQELPTALTKLVKEDEVV
SSEKAKDSFNEKRVAVEAPMREEYADFKPFERVWEVKDSKEDSDMLAAGGKIESNLESKV
DKKCFADSLEQTNHEKDSESSNDDTSFPSTPEGIKDRPGAYITCAPFNPAATESIATNIF
PLLGDPTSENKTDEKKIEEKKAQIVTEKNTSTKTSNPFLVAAQDSETDYVTTDNLTKVTE
EVVANMPEGLTPDLVQEACESELNEVTGTKIAYETKMDLVQTSEVMQESLYPAAQLCPSF
EESEATPSPVLPDIVMEAPLNSAVPSAGASVIQPSSSPLEASSVNYESIKHEPENPPPYE
EAMSVSLKKVSGIKEEIKEPENINAALQETEAPYISIACDLIKETKLSAEPAPDFSDYSE
MAKVEQPVPDHSELVEDSSPDSEPVDLFSDDSIPDVPQKQDETVMLVKESLTETSFESMI
EYENKEKLSALPPEGGKPYLESFKLSLDNTKDTLLPDEVSTLSKKEKIPLQMEELSTAVY
SNDDLFISKEAQIRETETFSDSSPIEIIDEFPTLISSKTDSFSKLAREYTDLEVSHKSEI
ANAPDGAGSLPCTELPHDLSLKNIQPKVEEKISFSDDFSKNGSATSKVLLLPPDVSALAT
QAEIESIVKPKVLVKEAEKKLPSDTEKEDRSPSAIFSAELSKTSVVDLLYWRDIKKTGVV
FGASLFLLLSLTVFSIVSVTAYIALALLSVTISFRIYKGVIQAIQKSDEGHPFRAYLESE
VAISEELVQKYSNSALGHVNCTIKELRRLFLVDDLVDSLKFAVLMWVFTYVGALFNGLTL

LILALISLFSVPVIYERHQAQIDHYLGLANKNVKDAMAKIQAKIPGLKRKAE

**SEQ ID NO:3**

GAAAATATGGACTTGAAGGAGCAGCCAGGTAACACTATTTCGGCTGGTCAAGAGGATTTC
CCATCTGTCCTGCTTGAAACTGCTGCTTCTNTTCCTTCTCTGTCTCCTCTCTCAGCCGCT
TCTTTCAAAGAACATGAATACCTTGGTAATTTGTCAACAGTATTACCCACTGAAGGAACA
CTTCAAGAAAATGTCAGTGAAGCTTCTAAAGAGGTCTCAGAGAAGGCAAAAACTCTACTC
ATAGATAGAGATTTAACAGAGTTTTCAGAATTAGAATACTCAGAAATGGGATCATCGTTC
AGTGTCTCTCCAAAAGCAGAATCTGCCGTAATAGTAGCAAATCCTAGGGAAGAAATAATC
GTGAAAAATAAAGATGAAGAAGAGAAGTTAGTTAGTAATAACATCCTTCATANTCAACAA
GAGTTACCTACAGCTCTTACTAAATTGGTTAAAGAGGATGAAGTTGTGTCTTCAGAAAAA
GCAAAAGACAGTTTTAATGAAAAGAGAGTTGCAGTGGAAGCTCCTATGAGGGAGGAATAT
GCAGACTTCAAACCATTTGAGCGAGTATGGGAAGTGAAAGATAGTAAGGAAGATAGTGAT
ATGTTGGCTGCTGGAGGTAAAATCGAGAGCAACTTGGAAAGTAAAGTGGATAAAAAATGT
TTTGCAGATAGCCTTGAGCAAACTAATCACGAAAAAGATAGTGAGAGTAGTAATGATGAT
ACTTCTTTCCCCAGTACGCCAGAAGGTATAAAGGATCGTTCAGGAGCATATATCACATGT
GCTCCCTTTAACCCAGCAGCAACTGAGAGCATTGCAACAAACATTTTTCCTTTGTTAGGA
GATCCTACTTCAGAAAATAAGACCGATG

**SEQ ID NO:4**

ENMDLKEQPGNTISAGQEDFPSVLLETAASXPSLSPLSAASFKEHEYLGNLSTVLPTEGT
LQENVSEASKEVSEKAKTLLIDRDLTEFSELEYSEMGSSFSVSPKAESAVIVANPREEII
VKNKDEEEKLVSNNILHXQQELPTALTKLVKEDEVVSSEKAKDSFNEKRVAVEAPMREEY
ADFKPFERVWEVKDSKEDSDMLAAGGKIESNLESKVDKKCFADSLEQTNHEKDSESSNDD
TSFPSTPEGIKDRSGAYITCAPFNPAATESIATNIFPLLGDPTSENKTD

**SEQ ID NO:5**

ATGGAAGACCTGGACCAGTCTCCTCTGGTCTCGTCCTCGGACAGCCCACCCCGGCCGCAG
CCCGCGTTCAAGTACCAGTTCGTGAGGGAGCCCGAGGACGAGGAGGAAGAAGAGGAGGAG
GAAGAGGAGGACGAGGACGAAGACCTGGAGGAGCTGGAGGTGCTGGAGAGGAAGCCCGCC
GCCGGGCTGTCCGCGGCCCCAGTGCCCACCGCCCCTGCCGCCGGCGCGCCCCTGATGGAC
TTCGGAAATGACTTCGTGCCGCCGGCGCCCCGGGGACCCCTGCCGGCCGCTCCCCCCGTC
GCCCCGGAGCGGCAGCCGTCTTGGGACCCGAGCCCGGTGTCGTCGACCGTGCCCGCGCCA
TCCCCGCTGTCTGCTGCCGCAGTCTCGCCCTCCAAGCTCCCTGAGGACGACGAGCCTCCG
GCCCGGCCTCCCCCTCCTCCCCCGGCCAGCGTGAGCCCCCAGGCAGAGCCCGTGTGGACC
CCGCCAGCCCCGGCTCCCGCCGCGCCCCCCTCCACCCCGGCCGCGCCCAAGCGCAGGGGC
TCCTCGGGCTCAGTGGTTGTTGACCTCCTGTACTGGAGAGACATTAAGAAGACTGGAGTG
GTGTTTGGTGCCAGCCTATTCCTGCTGCTTTCATTGACAGTATTCAGCATTGTGAGCGTA
ACAGCCTACATTGCCTTGGCCCTGCTCTCTGTGACCATCAGCTTTAGGATATACAAGGGT
GTGATCCAAGCTATCCAGAAATCAGATGAAGGCCACCCATTCAGGGCATATCTGGAATCT
GAAGTTGCTATATCTGAGGAGTTGGTTCAGAAGTACAGTAATTCTGCTCTTGGTCATGTG
AACTGCACGATAAAGGAACTCAGGCGCCTCTTCTTAGTTGATGATTTAGTTGATTCTCTG

AAGTTTGCAGTGTTGATGTGGGTATTTACCTATGTTGGTGCCTTGTTTAATGGTCTGACA
CTACTGATTTTGGCTCTCATTTCACTCTTCAGTGTTCCTGTTATTTATGAACGGCATCAG
GCACAGATAGATCATTATCTAGGACTTGCAAATAAGAATGTTAAAGATGCTATGGCTAAA
ATCCAAGCAAAAATCCCTGGATTGAAGCGCAAAGCTGAATGA

**SEQ ID NO:6**

MEDLDQSPLVSSSDSPPRPQPAFKYQFVREPEDEEEEEEEEEEDEDEDLEELEVLERKPA
AGLSAAPVPTAPAAGAPLMDFGNDFVPPAPRGPLPAAPPVAPERQPSWDPSPVSSTVPAP
SPLSAAAVSPSKLPEDDEPPARPPPPPPASVSPQAEPVWTPPAPAPAAPPSTPAAPKRRG
SSGSVVVDLLYWRDIKKTGVVFGASLFLLLSLTVFSIVSVTAYIALALLSVTISFRIYKG
VIQAIQKSDEGHPFRAYLESEVAISEELVQKYSNSALGHVNCTIKELRRLFLVDDLVDSL
KFAVLMWVFTYVGALFNGLTLLILALISLFSVPVIYERHQAQIDHYLGLANKNVKDAMAK
IQAKIPGLKRKAE

## Claims

1. An isolated polypeptide selected from the group consisting of:

   (a) an isolated polypeptide encoded by a polynucleotide comprising the sequence of SEQ ID NO:1 or SEQ ID NO:5;
   (b) an isolated polypeptide comprising a polypeptide sequence having at least 95% identity to the polypeptide sequence of SEQ ID NO:2 or SEQ ID NO:6;
   (c) an isolated polypeptide having at least 95% identity to the polypeptide sequence of SEQ ID NO:2 or SEQ ID NO:6; and
   (d) fragments and variants of such polypeptides in (a) to (e).

2. The isolated polypeptide as claimed in claim 1 comprising the polypeptide sequence of SEQ ID NO:2 or SEQ ID NO:6.

3. The isolated polypeptide as claimed in claim 1 which is the polypeptide sequence of SEQ ID NO:2 or SEQ ID NO:6.

4. An isolated polynucleotide selected from the group consisting of:

   (a) an isolated polynucleotide comprising a polynucleotide sequence having at least 95% identity to the polynucleotide sequence of SEQ ID NO:1 or SEQ ID NO:5;
   (b) an isolated polynucleotide having at least 95% identity to the polynucleotide of SEQ ID NO: 1 or SEQ ID NO:5;
   (c) an isolated polynucleotide comprising a polynucleotide sequence encoding a polypeptide sequence having at least 95% identity to the polypeptide sequence of SEQ ID NO:2 or SEQ ID NO:6;
   (d) an isolated polynucleotide having a polynucleotide sequence encoding a polypeptide sequence having at least 95% identity to the polypeptide sequence of SEQ ID NO:2 or SEQ ID NO:6;
   (e) an isolated polynucleotide with a nucleotide sequence of at least 100 nucleotidesobtained by screening a library under stringent hybridization conditions with a labeled probe having the sequence of SEQ ID NO: 1 or SEQ ID NO:5 or a fragment thereof having at least 15 nucleotides;
   (f) a polynucleotide which is the RNA equivalent of a polynucleotide of (a) to (e);
   or a polynucleotide sequence complementary to said isolated polynucleotide
   and polynucleotides that are variants and fragments of the above mentioned polynucleotides or that are complementary to above mentioned polynucleotides, over the entire length thereof.

5. An isolated polynucleotide as claimed in claim 4 selected from the group consisting of:

   (a) an isolated polynucleotide comprising the polynucleotide of SEQ ID NO:1 or SEQ ID NO:5;
   (b) the isolated polynucleotide of SEQ ID NO:1 or SEQ ID NO:5;
   (c) an isolated polynucleotide comprising a polynucleotide sequence encoding the polypeptide of SEQ ID NO: 2 or SEQ ID NO:6; and
   (d) an isolated polynucleotide encoding the polypeptide of SEQ ID NO:2 or SEQ ID NO:6.

6. An expression system comprising a polynucleotide capable of producing a polypeptide of claim 1 when said expression vector is present in a compatible host cell.

7. A recombinant host cell comprising the expression vector of claim 6 or a membrane thereof expressing the polypeptide of claim 1.

**8.** A process for producing a polypeptide of claim 1 comprising the step of culturing a host cell as defined in claim 7 under conditions sufficient for the production of said polypeptide and recovering the polypeptide from the culture medium.

**9.** An antibody immunospecific for the polypeptide of any one of claims 1 to 3.

**10.** A method for screening to identify compounds that stimulate or inhibit the function or level of the polypeptide of claim 1 comprising a method selected from the group consisting of:

(a) measuring or, detecting, quantitatively or qualitatively, the bindingof a candidate compound to the polypeptide (or to the cells or membranes expressing the polypeptide) or a fusion protein thereof by means of a label directly or indirectly associated with the candidate compound;
(b) measuring the competition of bindingof a candidate compound to the polypeptide (or to the cells or membranes expressing the polypeptide) or a fusion protein thereof in the presence of a labeled competitior;
(c) testing whether the candidate compound results in a signal generated by activation or inhibition of the polypeptide, using detection systems appropriate to the cells or cell membranes expressing the polypeptide;
(d) mixing a candidate compound with a solution containing a polypeptide of claim 1, to form a mixture, measuring activity of the polypeptide in the mixture, and comparing the activity of the mixture to a control mixture which contains no candidate compound; or
(e) detecting the effect of a candidate compound on the production ofmRNA encoding said polypeptide or said polypeptide in cells, using for instance, an ELISA assay.

**11.** An isolated polynucleotide selected form the group consisting of:

(a) an isolated polynucleotide comprising a nucleotide sequence which has at least 95% identity to SEQ ID NO: 3 over the entire length of SEQ ID NO:3;
(b) an isolated polynucleotide comprising the polynucleotide of SEQ ID NO:3; or
(c) the polynucleotide of SEQ ID NO:3.
(d) an isolated polynucleotide comprising a nucleotide sequence encoding a polypeptide which has at least 95% identity to the amino acid sequence of SEQ ID NO:4, over the entire length of SEQ ID NO:4.

**12.** A polypeptide selected from the group consisting of:

(a) a polypeptide which comprises an amino acid sequence which has at least 95% identity to that of SEQ ID NO:4 over the entire length of SEQ ID NO:4;
(b) a polypeptide in which the amino acid sequence has at least 95% identity to the amino acid sequence of SEQ ID NO:4 over the entire length of SEQ ID NO:4;
(c) a polypeptide which comprises the amino acid of SEQ ID NO:4;
(d) a polypeptide which is the polypeptide of SEQ ID NO:4; or
(e) a polypeptide which is encoded by a polynucleotide comprising the sequence contained in SEQ ID NO:3.

# Figure 1

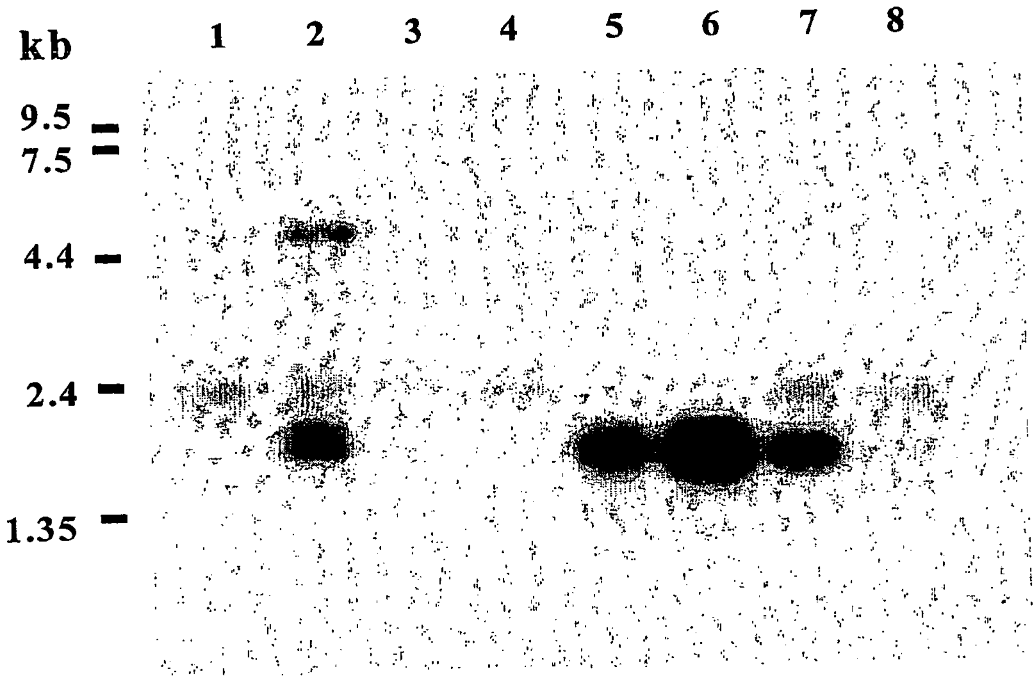

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4946778 A
- EP 0232262 A


### Non-patent literature cited in the description


- **J. Hens et al.** *Cell Tissue Res.,* 1998, vol. 292, 229-237
- **Geisler et al.** *Mamm. Genome,* 1998, vol. 9, 164-173
- **Spillmann et al.** *J. Biol. Chem.,* 1998, vol. 273, 15487-15493
- **Senden et al.** *Histochem. Cell Biol.,* 1997, vol. 108, 155-165
- **Adams, M.D. et al.** *Nature,* 1995, vol. 377, 3
- **Sambrook et al.** Molecular Cloning: A Laboratory Manual. Cold Spring Harbor Laboratory Press, 1989
- **Gentz et al.** *Proc Natl Acad Sci USA,* 1989, vol. 86, 821-824
- **Frohman et al.** *Proc Nat Acad Sci USA,* 1988, vol. 85, 8998-9002
- **Myers et al.** *Science,* 1985, vol. 230, 1242
- **Cotton et al.** *Proc Natl Acad Sci USA,* 1985, vol. 85, 4397-4401
- **M.Chee et al.** *Science,* 1996, vol. 274, 610-613
- **Walter, M. ; Spillett, D. ; Thomas, P. ; Weissenbach, J. ; Goodfellow, P.** A method for constructing radiation hybrid maps of whole genomes. *Nature Genetics,* 1994, vol. 7, 22-28
- **Gyapay G ; Schmitt K ; Fizames C ; Jones H ; Vega-Czarny N ; Spillett D ; Muselet D ; Prud'Homme JF ; Dib C ; Auffray C.** A radiation hybrid map of the human genome. *Hum Mol Genet,* March 1996, vol. 5 (3), 339-46
- **Schena et al.** *Science,* 1995, vol. 270, 467-470
- **Shalon et al.** *Genome Res,* 1996, vol. 6, 639-645
- **Kohler, G. ; Milstein, C.** *Nature,* 1975, vol. 256, 495-497
- **Kozbor et al.** *Immunology Today,* 1983, vol. 4, 72
- **Cole et al.** Monoclonal Antibodies and Cancer Therapy. Alan R. Liss, Inc, 1985, 77-96
- **Coligan et al.** Current Protocols in Immunology. 1991, vol. 1
- **Schullek et al.** *Anal Biochem.,* 1997, vol. 246, 20-29
- **D. Bennett et al.** *J Mol Recognition,* 1995, vol. 8, 52-58
- **K. Johanson et al.** *J Biol Chem,* 1995, vol. 270 (16), 9459-9471
- **T. E. Creighton.** Proteins - Structure and Molecular Properties. W. H. Freeman and Company, 1993
- Post-translational Protein Modifications: Perspectives and Prospects, 1-12. **Wold, F.** Post-translational Covalent Modification of Proteins. Academic Press, 1983
- **Seifter et al.** Analysis for protein modifications and nonprotein cofactors. *Meth Enzymol,* vol. 182, 626-646
- **Rattan et al.** Protein Synthesis: Post-translational Modifications and Aging. *Ann NY Acad Sci,* 1992, vol. 663, 48-62
- **Devereux J et al.** *Nucleic Acids Res,* 1984, vol. 12, 387-395
- **Smith ; Waterman.** *J Mol Biol,* 1981, vol. 147, 195-197
- *Advances in Applied Mathematics,* 1981, vol. 2, 482-489
- **Neddleman ; Wunsch.** *J Mol Biol,* 1970, vol. 48, 443-453
- **Altschul S F et al.** *J Mol Biol,* 1990, vol. 215, 403-410
- **Altschul S F et al.** *Nucleic Acids Res.,* 1997, vol. 25, 389-3402
- **Pearson W R.** *Methods in Enzymology,* 1990, vol. 183, 63-99
- **Pearson W R ; Lipman D J.** *Proc Nat Acad Sci USA,* 1998, vol. 85, 2444-2448
- **Henikoff S ; Henikoff J G.** *Proc. Nat. Acad Sci. USA,* 1992, vol. 89, 10915-10919

SEQUENCE LISTING

<110> SmithKline Beecham plc

<120> Novel compounds

<130> GP30165

<160> 6

<170> FastSEQ for Windows Version 3.0

<210> 1
<211> 3579
<212> DNA
<213> Homo sapiens

<400> 1
```
atggaagacc tggaccagtc tcctctggtc tcgtcctcgg acagcccacc ccggccgcag      60
cccgcgttca agtaccagtt cgtgagggag cccgaggacg aggaggaaga agaggaggag     120
gaagaggagg acgaggacga agacctggag gagctggagg tgctggagag gaagcccgcc     180
gccgggctgt ccgcggcccc agtgcccacc gcccctgccg ccggcgcgcc cctgatggac     240
ttcggaaatg acttcgtgcc gccggcgccc cggggacccc tgccggccgc tccccccgtc     300
gccccggagc ggcagccgtc ttgggacccg agcccggtgt cgtcgaccgt gcccgcgcca     360
tccccgctgt ctgctgccgc agtctcgccc tccaagctcc ctgaggacga cgagcctccg     420
gcccggcctc cccctcctcc cccggccagc gtgagccccc aggcagagcc cgtgtggacc     480
ccgccagccc cggctcccgc cgcgcccccc tccacccccgg ccgcgcccaa gcgcaggggc     540
tcctcgggct cagtggatga gacccttttt gctcttcctg ctgcatctga gcctgtgata     600
cgctcctctg cagaaaatat ggacttgaag gagcagccag gtaacactat ttcggctggt     660
caagaggatt tcccatctgt cctgcttgaa actgctgctt ctcttccttc tctgtctcct     720
ctctcagccg cttctttcaa agaacatgaa taccttggta atttgtcaac agtattaccc     780
actgaaggaa cacttcaaga aaatgtcagt gaagcttcta aagaggtctc agagaaggca     840
aaaactctac tcatagatag agatttaaca gagttttcag aattagaata ctcagaaatg     900
ggatcatcgt tcagtgtctc tccaaaagca gaatctgccg taatagtagc aaatcctagg     960
gaagaaataa tcgtgaaaaa taaagatgaa gaagagaagt tagttagtaa taacatcctt    1020
cataatcaac aagagttacc tacagctctt actaaattgg ttaaagagga tgaagttgtg    1080
tcttcagaaa aagcaaaaga cagttttaat gaaaagagag ttgcagtgga agctcctatg    1140
agggaggaat atgcagactt caaaccattt gagcgagtat gggaagtgaa agatagtaag    1200
gaagatagtg atatgttggc tgctggaggt aaaatcgaga gcaacttgga aagtaaagtg    1260
gataaaaaat gttttgcaga tagccttgag caaactaatc acgaaaaaga tagtgagagt    1320
```

agtaatgatg atacttcttt ccccagtacg ccagaaggta taaaggatcg tccaggagca 1380
tatatcacat gtgctccctt taacccagca gcaactgaga gcattgcaac aaacattttt 1440
cctttgttag gagatcctac ttcagaaaat aagaccgatg aaaaaaaaat agaagaaaag 1500
aaggcccaaa tagtaacaga gaagaatact agcaccaaaa catcaaaccc ttttcttgta 1560
gcagcacagg attctgagac agattatgtc acaacagata atttaacaaa ggtgactgag 1620
gaagtcgtgg caaacatgcc tgaaggcctg actccagatt tagtacagga agcatgtgaa 1680
agtgaattga atgaagttac tggtacaaag attgcttatg aaacaaaaat ggacttggtt 1740
caaacatcag aagttatgca agagtcactc tatcctgcag cacagctttg cccatcattt 1800
gaagagtcag aagctactcc ttcaccagtt ttgcctgaca ttgttatgga agcaccattg 1860
aattctgcag ttcctagtgc tggtgcttcc gtgatacagc ccagctcatc accattagaa 1920
gcttcttcag ttaattatga aagcataaaa catgagcctg aaaacccccc accatatgaa 1980
gaggccatga gtgtatcact aaaaaaagta tcaggaataa aggaagaaat taaagagcct 2040
gaaaatatta atgcagctct tcaagaaaca gaagctcctt atatatctat tgcatgtgat 2100
ttaattaaag aaacaaagct ttctgctgaa ccagctccgg atttctctga ttattcagaa 2160
atggcaaaag ttgaacagcc agtgcctgat cattctgagc tagttgaaga ttcctcacct 2220
gattctgaac cagttgactt atttagtgat gattcaatac ctgacgttcc acaaaaacaa 2280
gatgaaactg tgatgcttgt gaaagaaagt ctcactgaga cttcatttga gtcaatgata 2340
gaatatgaaa ataaggaaaa actcagtgct ttgccacctg agggaggaaa gccatatttg 2400
gaatctttta agctcagttt agataacaca aaagataccc tgttacctga tgaagtttca 2460
acattgagca aaaaggagaa aattcctttg cagatggagg agctcagtac tgcagtttat 2520
tcaaatgatg acttatttat ttctaaggaa gcacagataa gagaaactga aacgttttca 2580
gattcatctc caattgaaat tatagatgag ttccctacat tgatcagttc taaaactgat 2640
tcattttcta aattagccag ggaatatact gacctagaag tatcccacaa aagtgaaatt 2700
gctaatgccc cggatggagc tgggtcattg ccttgcacag aattgcccca tgacctttct 2760
ttgaagaaca tacaacccaa agttgaagag aaaatcagtt tctcagatga cttttctaaa 2820
aatgggtctg ctacatcaaa ggtgctctta ttgcctccag atgtttctgc tttggccact 2880
caagcagaga tagagagcat agttaaaccc aaagttcttg tgaaagaagc tgagaaaaaa 2940
cttccttccg atacagaaaa agaggacaga tcaccatctg ctatattttc agcagagctg 3000
agtaaaactt cagttgttga cctcctgtac tggagagaca ttaagaagac tggagtggtg 3060
tttggtgcca gcctattcct gctgctttca ttgacagtat tcagcattgt gagcgtaaca 3120
gcctacattg ccttggccct gctctctgtg accatcagct ttaggatata caagggtgtg 3180
atccaagcta tccagaaatc agatgaaggc cacccattca gggcatatct ggaatctgaa 3240
gttgctatat ctgaggagtt ggttcagaag tacagtaatt ctgctcttgg tcatgtgaac 3300
tgcacgataa aggaactcag gcgcctcttc ttagttgatg atttagttga ttctctgaag 3360
tttgcagtgt tgatgtgggt atttacctat gttggtgcct tgtttaatgg tctgacacta 3420
ctgattttgg ctctcatttc actcttcagt gttcctgtta tttatgaacg gcatcaggcg 3480
cagatagatc attatctagg acttgcaaat aagaatgtta aagatgctat ggctaaaatc 3540
caagcaaaaa tccctggatt gaagcgcaaa gctgaatga 3579

<210> 2
<211> 1192
<212> PRT

<213> Homo sapiens

<400> 2

Met Glu Asp Leu Asp Gln Ser Pro Leu Val Ser Ser Ser Asp Ser Pro
1 5 10 15

Pro Arg Pro Gln Pro Ala Phe Lys Tyr Gln Phe Val Arg Glu Pro Glu
20 25 30

Asp Glu Glu Glu Glu Glu Glu Glu Glu Glu Glu Asp Glu Asp Glu Asp
35 40 45

Leu Glu Glu Leu Glu Val Leu Glu Arg Lys Pro Ala Ala Gly Leu Ser
50 55 60

Ala Ala Pro Val Pro Thr Ala Pro Ala Ala Gly Ala Pro Leu Met Asp
65 70 75 80

Phe Gly Asn Asp Phe Val Pro Pro Ala Pro Arg Gly Pro Leu Pro Ala
85 90 95

Ala Pro Pro Val Ala Pro Glu Arg Gln Pro Ser Trp Asp Pro Ser Pro
100 105 110

Val Ser Ser Thr Val Pro Ala Pro Ser Pro Leu Ser Ala Ala Ala Val
115 120 125

Ser Pro Ser Lys Leu Pro Glu Asp Asp Glu Pro Pro Ala Arg Pro Pro
130 135 140

Pro Pro Pro Pro Ala Ser Val Ser Pro Gln Ala Glu Pro Val Trp Thr
145 150 155 160

Pro Pro Ala Pro Ala Pro Ala Ala Pro Pro Ser Thr Pro Ala Ala Pro
165 170 175

Lys Arg Arg Gly Ser Ser Gly Ser Val Asp Glu Thr Leu Phe Ala Leu
180 185 190

Pro Ala Ala Ser Glu Pro Val Ile Arg Ser Ser Ala Glu Asn Met Asp
195 200 205

Leu Lys Glu Gln Pro Gly Asn Thr Ile Ser Ala Gly Gln Glu Asp Phe
210 215 220

Pro Ser Val Leu Leu Glu Thr Ala Ala Ser Leu Pro Ser Leu Ser Pro
225 230 235 240

Leu Ser Ala Ala Ser Phe Lys Glu His Glu Tyr Leu Gly Asn Leu Ser
245 250 255

Thr Val Leu Pro Thr Glu Gly Thr Leu Gln Glu Asn Val Ser Glu Ala
260 265 270

Ser Lys Glu Val Ser Glu Lys Ala Lys Thr Leu Leu Ile Asp Arg Asp
275 280 285

Leu Thr Glu Phe Ser Glu Leu Glu Tyr Ser Glu Met Gly Ser Ser Phe
290 295 300

Ser Val Ser Pro Lys Ala Glu Ser Ala Val Ile Val Ala Asn Pro Arg

305 310 315 320
Glu Glu Ile Ile Val Lys Asn Lys Asp Glu Glu Glu Lys Leu Val Ser
325 330 335
Asn Asn Ile Leu His Asn Gln Gln Glu Leu Pro Thr Ala Leu Thr Lys
340 345 350
Leu Val Lys Glu Asp Glu Val Val Ser Ser Glu Lys Ala Lys Asp Ser
355 360 365
Phe Asn Glu Lys Arg Val Ala Val Glu Ala Pro Met Arg Glu Glu Tyr
370 375 380
Ala Asp Phe Lys Pro Phe Glu Arg Val Trp Glu Val Lys Asp Ser Lys
385 390 395 400
Glu Asp Ser Asp Met Leu Ala Ala Gly Gly Lys Ile Glu Ser Asn Leu
405 410 415
Glu Ser Lys Val Asp Lys Lys Cys Phe Ala Asp Ser Leu Glu Gln Thr
420 425 430
Asn His Glu Lys Asp Ser Glu Ser Ser Asn Asp Asp Thr Ser Phe Pro
435 440 445
Ser Thr Pro Glu Gly Ile Lys Asp Arg Pro Gly Ala Tyr Ile Thr Cys
450 455 460
Ala Pro Phe Asn Pro Ala Ala Thr Glu Ser Ile Ala Thr Asn Ile Phe
465 470 475 480
Pro Leu Leu Gly Asp Pro Thr Ser Glu Asn Lys Thr Asp Glu Lys Lys
485 490 495
Ile Glu Glu Lys Lys Ala Gln Ile Val Thr Glu Lys Asn Thr Ser Thr
500 505 510
Lys Thr Ser Asn Pro Phe Leu Val Ala Ala Gln Asp Ser Glu Thr Asp
515 520 525
Tyr Val Thr Thr Asp Asn Leu Thr Lys Val Thr Glu Glu Val Val Ala
530 535 540
Asn Met Pro Glu Gly Leu Thr Pro Asp Leu Val Gln Glu Ala Cys Glu
545 550 555 560
Ser Glu Leu Asn Glu Val Thr Gly Thr Lys Ile Ala Tyr Glu Thr Lys
565 570 575
Met Asp Leu Val Gln Thr Ser Glu Val Met Gln Glu Ser Leu Tyr Pro
580 585 590
Ala Ala Gln Leu Cys Pro Ser Phe Glu Glu Ser Glu Ala Thr Pro Ser
595 600 605
Pro Val Leu Pro Asp Ile Val Met Glu Ala Pro Leu Asn Ser Ala Val
610 615 620
Pro Ser Ala Gly Ala Ser Val Ile Gln Pro Ser Ser Ser Pro Leu Glu
625 630 635 640
Ala Ser Ser Val Asn Tyr Glu Ser Ile Lys His Glu Pro Glu Asn Pro

645 650 655

Pro Pro Tyr Glu Glu Ala Met Ser Val Ser Leu Lys Lys Val Ser Gly

660 665 670

Ile Lys Glu Glu Ile Lys Glu Pro Glu Asn Ile Asn Ala Ala Leu Gln

675 680 685

Glu Thr Glu Ala Pro Tyr Ile Ser Ile Ala Cys Asp Leu Ile Lys Glu

690 695 700

Thr Lys Leu Ser Ala Glu Pro Ala Pro Asp Phe Ser Asp Tyr Ser Glu

705 710 715 720

Met Ala Lys Val Glu Gln Pro Val Pro Asp His Ser Glu Leu Val Glu

725 730 735

Asp Ser Ser Pro Asp Ser Glu Pro Val Asp Leu Phe Ser Asp Asp Ser

740 745 750

Ile Pro Asp Val Pro Gln Lys Gln Asp Glu Thr Val Met Leu Val Lys

755 760 765

Glu Ser Leu Thr Glu Thr Ser Phe Glu Ser Met Ile Glu Tyr Glu Asn

770 775 780

Lys Glu Lys Leu Ser Ala Leu Pro Pro Glu Gly Gly Lys Pro Tyr Leu

785 790 795 800

Glu Ser Phe Lys Leu Ser Leu Asp Asn Thr Lys Asp Thr Leu Leu Pro

805 810 815

Asp Glu Val Ser Thr Leu Ser Lys Lys Glu Lys Ile Pro Leu Gln Met

820 825 830

Glu Glu Leu Ser Thr Ala Val Tyr Ser Asn Asp Asp Leu Phe Ile Ser

835 840 845

Lys Glu Ala Gln Ile Arg Glu Thr Glu Thr Phe Ser Asp Ser Ser Pro

850 855 860

Ile Glu Ile Ile Asp Glu Phe Pro Thr Leu Ile Ser Ser Lys Thr Asp

865 870 875 880

Ser Phe Ser Lys Leu Ala Arg Glu Tyr Thr Asp Leu Glu Val Ser His

885 890 895

Lys Ser Glu Ile Ala Asn Ala Pro Asp Gly Ala Gly Ser Leu Pro Cys

900 905 910

Thr Glu Leu Pro His Asp Leu Ser Leu Lys Asn Ile Gln Pro Lys Val

915 920 925

Glu Glu Lys Ile Ser Phe Ser Asp Asp Phe Ser Lys Asn Gly Ser Ala

930 935 940

Thr Ser Lys Val Leu Leu Leu Pro Pro Asp Val Ser Ala Leu Ala Thr

945 950 955 960

Gln Ala Glu Ile Glu Ser Ile Val Lys Pro Lys Val Leu Val Lys Glu

965 970 975

Ala Glu Lys Lys Leu Pro Ser Asp Thr Glu Lys Glu Asp Arg Ser Pro

980 985 990

Ser Ala Ile Phe Ser Ala Glu Leu Ser Lys Thr Ser Val Val Asp Leu

995 1000 1005

Leu Tyr Trp Arg Asp Ile Lys Lys Thr Gly Val Val Phe Gly Ala Ser

1010 1015 1020

Leu Phe Leu Leu Leu Ser Leu Thr Val Phe Ser Ile Val Ser Val Thr

1025 1030 1035 104

Ala Tyr Ile Ala Leu Ala Leu Leu Ser Val Thr Ile Ser Phe Arg Ile

1045 1050 1055

Tyr Lys Gly Val Ile Gln Ala Ile Gln Lys Ser Asp Glu Gly His Pro

1060 1065 1070

Phe Arg Ala Tyr Leu Glu Ser Glu Val Ala Ile Ser Glu Glu Leu Val

1075 1080 1085

Gln Lys Tyr Ser Asn Ser Ala Leu Gly His Val Asn Cys Thr Ile Lys

1090 1095 1100

Glu Leu Arg Arg Leu Phe Leu Val Asp Asp Leu Val Asp Ser Leu Lys

1105 1110 1115 112

Phe Ala Val Leu Met Trp Val Phe Thr Tyr Val Gly Ala Leu Phe Asn

1125 1130 1135

Gly Leu Thr Leu Leu Ile Leu Ala Leu Ile Ser Leu Phe Ser Val Pro

1140 1145 1150

Val Ile Tyr Glu Arg His Gln Ala Gln Ile Asp His Tyr Leu Gly Leu

1155 1160 1165

Ala Asn Lys Asn Val Lys Asp Ala Met Ala Lys Ile Gln Ala Lys Ile

1170 1175 1180

Pro Gly Leu Lys Arg Lys Ala Glu

1185 1190

<210> 3

<211> 868

<212> DNA

<213> Homo sapiens

<400> 3

gaaaatatgg acttgaagga gcagccaggt aacactattt cggctggtca agaggatttc 60

ccatctgtcc tgcttgaaac tgctgcttct nttccttctc tgtctcctct ctcagccgct 120

tctttcaaag aacatgaata ccttggtaat ttgtcaacag tattacccac tgaaggaaca 180

cttcaagaaa atgtcagtga agcttctaaa gaggtctcag agaaggcaaa aactctactc 240

atagatagag atttaacaga gttttcagaa ttagaatact cagaaatggg atcatcgttc 300

agtgtctctc caaaagcaga atctgccgta atagtagcaa atcctaggga agaaataatc 360

gtgaaaaata aagatgaaga agagaagtta gttagtaata acatccttca tantcaacaa 420

gagttaccta cagctcttac taaattggtt aaagaggatg aagttgtgtc ttcagaaaaa 480

gcaaaagaca gttttaatga aaagagagtt gcagtggaag ctcctatgag ggaggaatat 540
gcagacttca aaccatttga gcgagtatgg gaagtgaaag atagtaagga agatagtgat 600
atgttggctg ctggaggtaa aatcgagagc aacttggaaa gtaaagtgga taaaaaatgt 660
tttgcagata gccttgagca aactaatcac gaaaaagata gtgagagtag taatgatgat 720
acttctttcc ccagtacgcc agaaggtata aaggatcgtt caggagcata tatcacatgt 780
gctcccttta acccagcagc aactgagagc attgcaacaa acatttttcc tttgttagga 840
gatcctactt cagaaaataa gaccgatg 868

<210> 4
<211> 289
<212> PRT
<213> Homo sapiens

<400> 4

Glu Asn Met Asp Leu Lys Glu Gln Pro Gly Asn Thr Ile Ser Ala Gly
1 5 10 15
Gln Glu Asp Phe Pro Ser Val Leu Leu Glu Thr Ala Ala Ser Xaa Pro
20 25 30
Ser Leu Ser Pro Leu Ser Ala Ala Ser Phe Lys Glu His Glu Tyr Leu
35 40 45
Gly Asn Leu Ser Thr Val Leu Pro Thr Glu Gly Thr Leu Gln Glu Asn
50 55 60
Val Ser Glu Ala Ser Lys Glu Val Ser Glu Lys Ala Lys Thr Leu Leu
65 70 75 80
Ile Asp Arg Asp Leu Thr Glu Phe Ser Glu Leu Glu Tyr Ser Glu Met
85 90 95
Gly Ser Ser Phe Ser Val Ser Pro Lys Ala Glu Ser Ala Val Ile Val
100 105 110
Ala Asn Pro Arg Glu Glu Ile Ile Val Lys Asn Lys Asp Glu Glu Glu
115 120 125
Lys Leu Val Ser Asn Asn Ile Leu His Xaa Gln Gln Glu Leu Pro Thr
130 135 140
Ala Leu Thr Lys Leu Val Lys Glu Asp Glu Val Val Ser Ser Glu Lys
145 150 155 160
Ala Lys Asp Ser Phe Asn Glu Lys Arg Val Ala Val Glu Ala Pro Met
165 170 175
Arg Glu Glu Tyr Ala Asp Phe Lys Pro Phe Glu Arg Val Trp Glu Val
180 185 190
Lys Asp Ser Lys Glu Asp Ser Asp Met Leu Ala Ala Gly Gly Lys Ile
195 200 205
Glu Ser Asn Leu Glu Ser Lys Val Asp Lys Lys Cys Phe Ala Asp Ser
210 215 220

Leu Glu Gln Thr Asn His Glu Lys Asp Ser Glu Ser Ser Asn Asp Asp
225 230 235 240
Thr Ser Phe Pro Ser Thr Pro Glu Gly Ile Lys Asp Arg Ser Gly Ala
245 250 255
Tyr Ile Thr Cys Ala Pro Phe Asn Pro Ala Ala Thr Glu Ser Ile Ala
260 265 270
Thr Asn Ile Phe Pro Leu Leu Gly Asp Pro Thr Ser Glu Asn Lys Thr
275 280 285
Asp

<210> 5
<211> 1122
<212> DNA
<213> Homo sapiens

<400> 5
atggaagacc tggaccagtc tcctctggtc tcgtcctcgg acagcccacc ccggccgcag 60
cccgcgttca agtaccagtt cgtgagggag cccgaggacg aggaggaaga agaggaggag 120
gaagaggagg acgaggacga agacctggag gagctggagg tgctggagag gaagcccgcc 180
gccgggctgt ccgcggcccc agtgcccacc gcccctgccg ccggcgcgcc cctgatggac 240
ttcggaaatg acttcgtgcc gccggcgccc cggggacccc tgccggccgc tccccccgtc 300
gccccggagc ggcagccgtc ttgggacccg agcccggtgt cgtcgaccgt gcccgcgcca 360
tccccgctgt ctgctgccgc agtctcgccc tccaagctcc ctgaggacga cgagcctccg 420
gcccggcctc cccctcctcc cccggccagc gtgagccccc aggcagagcc cgtgtggacc 480
ccgccagccc cggctcccgc cgcgcccccc tccacccogg ccgcgcccaa gcgcaggggc 540
tcctcgggct cagtggttgt tgacctcctg tactggagag acattaagaa gactggagtg 600
gtgtttggtg ccagcctatt cctgctgctt tcattgacag tattcagcat tgtgagcgta 660
acagcctaca ttgccttggc cctgctctct gtgaccatca gctttaggat atacaagggt 720
gtgatccaag ctatccagaa atcagatgaa ggccacccat tcagggcata tctggaatct 780
gaagttgcta tatctgagga gttggttcag aagtacagta attctgctct tggtcatgtg 840
aactgcacga taaaggaact caggcgcctc ttcttagttg atgatttagt tgattctctg 900
aagtttgcag tgttgatgtg ggtatttacc tatgttggtg ccttgtttaa tggtctgaca 960
ctactgattt tggctctcat ttcactcttc agtgttcctg ttatttatga acggcatcag 1020
gcacagatag atcattatct aggacttgca aataagaatg ttaaagatgc tatggctaaa 1080
atccaagcaa aaatccctgg attgaagcgc aaagctgaat ga 1122

<210> 6
<211> 373
<212> PRT
<213> Homo sapiens

<400> 6

Met Glu Asp Leu Asp Gln Ser Pro Leu Val Ser Ser Ser Asp Ser Pro
1               5                   10                  15

Pro Arg Pro Gln Pro Ala Phe Lys Tyr Gln Phe Val Arg Glu Pro Glu
            20                  25                  30

Asp Glu Glu Glu Glu Glu Glu Glu Glu Glu Glu Asp Glu Asp Glu Asp
        35                  40                  45

Leu Glu Glu Leu Glu Val Leu Glu Arg Lys Pro Ala Ala Gly Leu Ser
    50                  55                  60

Ala Ala Pro Val Pro Thr Ala Pro Ala Ala Gly Ala Pro Leu Met Asp
65                  70                  75                  80

Phe Gly Asn Asp Phe Val Pro Pro Ala Pro Arg Gly Pro Leu Pro Ala
                85                  90                  95

Ala Pro Pro Val Ala Pro Glu Arg Gln Pro Ser Trp Asp Pro Ser Pro
            100                 105                 110

Val Ser Ser Thr Val Pro Ala Pro Ser Pro Leu Ser Ala Ala Ala Val
        115                 120                 125

Ser Pro Ser Lys Leu Pro Glu Asp Asp Glu Pro Pro Ala Arg Pro Pro
    130                 135                 140

Pro Pro Pro Pro Ala Ser Val Ser Pro Gln Ala Glu Pro Val Trp Thr
145                 150                 155                 160

Pro Pro Ala Pro Ala Pro Ala Ala Pro Pro Ser Thr Pro Ala Ala Pro
                165                 170                 175

Lys Arg Arg Gly Ser Ser Gly Ser Val Val Val Asp Leu Leu Tyr Trp
            180                 185                 190

Arg Asp Ile Lys Lys Thr Gly Val Val Phe Gly Ala Ser Leu Phe Leu
        195                 200                 205

Leu Leu Ser Leu Thr Val Phe Ser Ile Val Ser Val Thr Ala Tyr Ile
    210                 215                 220

Ala Leu Ala Leu Leu Ser Val Thr Ile Ser Phe Arg Ile Tyr Lys Gly
225                 230                 235                 240

Val Ile Gln Ala Ile Gln Lys Ser Asp Glu Gly His Pro Phe Arg Ala
                245                 250                 255

Tyr Leu Glu Ser Glu Val Ala Ile Ser Glu Glu Leu Val Gln Lys Tyr
            260                 265                 270

Ser Asn Ser Ala Leu Gly His Val Asn Cys Thr Ile Lys Glu Leu Arg
        275                 280                 285

Arg Leu Phe Leu Val Asp Asp Leu Val Asp Ser Leu Lys Phe Ala Val
    290                 295                 300

Leu Met Trp Val Phe Thr Tyr Val Gly Ala Leu Phe Asn Gly Leu Thr
305                 310                 315                 320

Leu Leu Ile Leu Ala Leu Ile Ser Leu Phe Ser Val Pro Val Ile Tyr

325 330 335
Glu Arg His Gln Ala Gln Ile Asp His Tyr Leu Gly Leu Ala Asn Lys
340 345 350
Asn Val Lys Asp Ala Met Ala Lys Ile Gln Ala Lys Ile Pro Gly Leu
355 360 365
Lys Arg Lys Ala Glu
370